# EUROPEAN PATENT SPECIFICATION

(11) **EP 1 496 361 B1**
(45) Date of publication and mention of the grant of the patent: **25.10.2006**
(21) Application number: 04016024.4
(22) Date of filing: 07.07.2004
(51) Int. Cl.: G01N 33/53

(54) **Immunoassay method and immunoassay system using a Fourier transformation to judge the occurrence of zone phenomena.**
Immuntest - Verfahren und Vorrichtung, welche zum Nachweis von Zonenphänomenen dient unter Verwendung einer Fourier-Transformation.
Assay et système immunologique, utilisant une transformation Fourier, pour évaluer la survenue des phénomènes de zones.

(30) Priority: 07.07.2003 JP 2003271423
(43) Date of publication of application: 12.01.2005
(73) Proprietor: MATSUSHITA ELECTRIC INDUSTRIAL CO., LTD., Kadoma-shi, Osaka 571-8501 (JP)
(72) Inventor: Kamei, Akihito, Yawata-shi Kyoto 614-8295 (JP); Kawamura, Tatsurou, Kyotanabe-shi Kyoto 610-0351 (JP)
(74) Representative: Grünecker, Kinkeldey, Stockmair & Schwanhäusser Anwaltssozietät

(56) References cited:
- EP-A- 0 433 629
- EP-A- 0 990 901
- EP-A- 1 293 780
- WO-A-91/04489
- US-A1- 2003 100 128
- PATENT ABSTRACTS OF JAPAN vol. 1999, no. 14, 22 December 1999 (1999-12-22) & JP 11 264821 A (OLYMPUS OPTICAL CO LTD), 28 September 1999 (1999-09-28)
- PATENT ABSTRACTS OF JAPAN vol. 1998, no. 09, 31 July 1998 (1998-07-31) & JP 10 090268 A (EIKEN CHEM CO LTD), 10 April 1998 (1998-04-10)
- PATENT ABSTRACTS OF JAPAN vol. 2003, no. 09, 3 September 2003 (2003-09-03) & JP 2003 149244 A (AZWELL INC), 21 May 2003 (2003-05-21)
- PATENT ABSTRACTS OF JAPAN vol. 2000, no. 11, 3 January 2001 (2001-01-03) & JP 2000 221195 A (TOSHIBA IYO SYSTEM ENGINEERING KK; TOSHIBA CORP), 11 August 2000 (2000-08-11)
- PATENT ABSTRACTS OF JAPAN vol. 012, no. 226 (P-722), 28 June 1988 (1988-06-28) & JP 63 019560 A (SHIMADZU CORP), 27 January 1988 (1988-01-27)

## Description

### BACKGROUND OF THE INVENTION

The present invention relates to an immunoassay method which allows measurement of the content of a substance to be measured in a sample and an immunoassay system used in the immunoassay method.

In the field of clinical examination, for diagnosis of various diseases and examination of progress of the condition of a disease, measurement of the content of a characteristic protein distinctive to each disease existing in human body fluids has been widely used. For such measurement of protein content, mainly, immunoassay utilizing a reaction (antigen-antibody reaction) employing an antibody capable of specifically recognizing a target protein as an antigen has been widely used. At present, immunoassay methods based on various different principles have been developed.

Among such immunoassay methods, measurement methods such as immunonephelometry (which will be hereinafter referred to as a "nephelometry"), immunoturbidimetry (which will be hereinafter referred to as a "turbidimetry") and a slide agglutination, used for detection of agglutinates of antigen-antibody complexes (which will be hereinafter referred to as "agglutinate complexes") generated due to an antigen-antibody reaction for the purpose of measurement of the content of a substance to be measured in a sample, have been well known. In an antigen-antibody reaction, turbidity occurs in a reaction system due to the generation of agglutinate complexes. The degree of the turbidity generated due to the generation of agglutinate complexes depends on the amounts of an antigen and an antibody. Nephelometry and turbidimetry are methods, based on this principle, for optically measuring the degree of turbidity generated in a reaction system and calculating the amount of an antigen or an antibody from an obtained measurement value. Specifically, nephelometry is a method for measuring agglutinate complexes based on a change in the amount of light scattered around in a reaction system and turbidimetry is a method for measuring agglutinate complexes based on a change in a reduction amount of the amount of transmitted light due to scatter of light in a reaction system. These measurement methods are used for measurement performed with antigens and antibodies uniformly dispersed. Therefore, the measurements are generically called "homogeneous immunoassay".

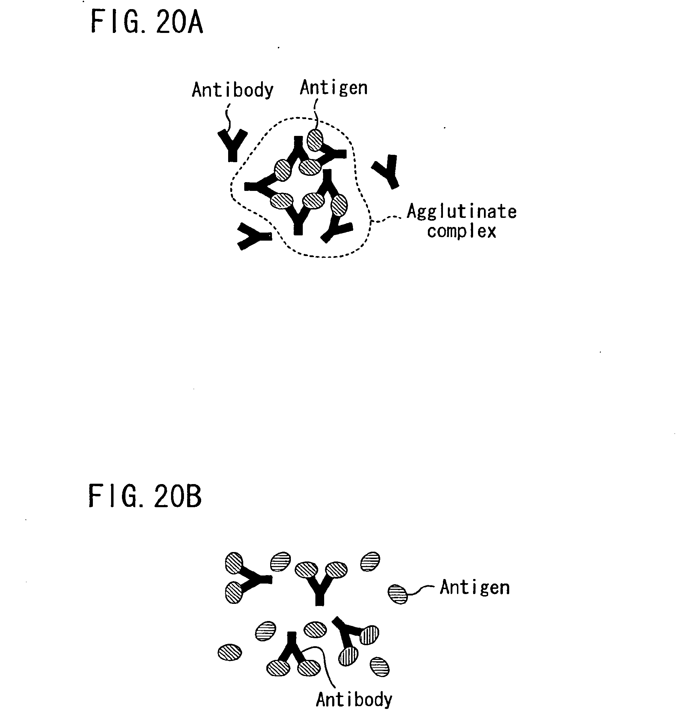

In general, it has been known that in a homogeneous immunoassay method, a phenomenon called "zone phenomenon" occurs. The zone phenomenon is a phenomenon where when an excessive existence of antigens or antibodies, compared to an equivalent point in which the antigens and the antibody form the maximum amount of agglutinate complexes, is observed, agglutinate complexes are hardly generated. Especially, antigen excess causes problems in many cases. Hereinafter, this phenomenon will be described with reference to FIGS. **20A** and **20B**. FIGS. **20A** and **20B** are schematic views illustrating a reaction of antigens and antibodies in a reaction system used in homogeneous immunoassay.

As shown in FIG. **20A**, normally in homogeneous immunoassay, an agglutinate complex as a huge molecular chain including an antibody and an antigen alternately binding to each other is formed. The zone phenomenon is a phenomenon where an agglutinate complex is less easily formed when the antigen exists in an excessive amount with respect to the antibody, as shown in FIG. **20B**, compared to the amount in an equivalent point in which the antigen and the antibody form the maximum amount of agglutinate complexes. As for the binding reaction between a multivalent antibody and a divalent or higher-valent antigen, the lattice theory by Heidelberger et al., is famous and details are described in William E. Paul, Fundamental Immunology, 1989.

In an antigen/antibody concentration range free from the zone phenomenon, as shown in FIG. **20A**, an agglutinate complex is formed as a large molecule chain in which the antibody and the antigen alternately bind to each other. If in this case, an antibody concentration is constant, the amount and size of the agglutinate complexes is increased with increase of the antigen concentration. Therefore, by measuring each of the amount and size of the agglutinate complexes as an optical change, the antigen concentration can be quantitatively measured. Moreover, the agglutinate complexes can be visibly confirmed by the naked eyes as turbidity or agglutinates, depending on the antibody and antigen concentrations. Therefore, it is also possible to make qualitative judgment by visual observation and the like.

However, in actual homogeneous immunoassay, an antigen concentration is often measured using an antibody. Moreover, measurement values often have significant meanings in the case where the antigen concentration is high, compared to the case where it is low. However, when the antigen exists in an excessive amount with respect to the antibody, the amount of the antibody with binding sites saturated by the antigen is increased, as shown in FIG. **20B**, and thus the generation of agglutinate complexes becomes difficult. Therefore, it is difficult to distinguish results of the antigen-antibody reaction in the case where the antigen concentration is low from those in the case where the antigen is excessive. Accordingly, correct quantification and judgment according to the antigen concentration may not be attained. In another case, to perform qualification and judgment according to the antigen concentration, a measurable concentration range is limited. As described above, the zone phenomenon caused by the antigen excess often becomes a cause of a problem in homogeneous immunoassay.

To solve the above-described problems, disclosed is a method in which an antibody (or antigen), i.e., a binding substance capable of specifically binding to an antigen (or antibody), i.e., a substance to be measured is prepared at different concentrations at which the content of the antibody (or antigen) is 1/n (where n is an arbitrary number larger than 1), the binding substance at each concentration is brought into reaction with the subject substance and then a generated agglutinate complex is measured (see Japanese Laid-Open Publication No. 60-79269). According to the method, whether or not an antigen-antibody reaction has occurred within a concentration range in which antigen excess is present (which will be hereinafter referred to as a "zone region" can be judged.

Moreover, a method in which from measurement results obtained by measurement using two lights having different wavelengths, a judgment index such as absorbency ratio is obtained by calculation and whether or not an antigen-antibody reaction has occurred in a zone region is judged based on the judgment index is disclosed (see Japanese Laid-Open Publication No. 63-19560, Japanese Laid-Open Publication No. 63-149564 and Japanese Laid-Open Publication No. 4-204378).

Furthermore, a method in which using a reaction rate obtained by differentiation of changes with time of an optical signal to be measured and whether or not an antigen-antibody reaction has been occurred in a zone region is judged based on a peak rate value at which the reaction rate is at a maximum level and an elapsed time from a start of a reaction to the time when a peak rate is reached is disclosed (see United States Patent No. 4,157,871).

However, the method disclosed in Japanese Laid-Open Publication No. 60-79269, a plurality of reaction vessels have to be prepared and a plurality number of measurements have to be performed for each sample. Accordingly, measurement procedures become complicated.

Moreover, in the methods disclosed in Japanese Laid-Open Publication No. 63-19560, Japanese Laid-Open Publication No. 63-149564 and Japanese Laid-Open Publication No. 4-204378, a plurality of light sources have to be prepared, or means for separating light at each wavelength from white light and means for performing a plurality number of measurements or means for performing wavelength separation in a detector side have to be provided. Accordingly, the configuration of a measurement system becomes complex and also measurement procedures become complicated.

Furthermore, in the method disclosed in United States Patent No. 4,157,871, when the peak rate value is small, the peak rate value is easily influenced by a contaminant such as bubbles and dusts, and the generation of noise due to influence of a magnetic waves on a detector and the like. Therefore, the possibility of the occurrence of an error in judgment results is increased.

The US application 2003/0100129 relates to a specific binding analysis method of quantitatively determining an analyte in a sample from a signal attributed to a specific binding reaction between the analyte in the sample and a specific binding substance capable of specifically binding to the analyte, characterized by comprising the steps of: (a) previously preparing a database comprising, for an individual sample containing a suspected analyte, at least the relation between a concentration of the analyte and a saturation value or change of a signal intensity attributed to a specific binding reaction between the analyte in the sample and a specific binding substance capable of specifically binding to the analyte, the change over time of the signal intensity, and time tₛ at which the saturation value is obtained and time tₘ at which a maximum value of the signal intensity is obtained; (b) preparing a sample containing an analyte and determining, based on the database, a measurement pattern of the signal intensity corresponding to the sample, (c) causing a specific binding reaction between the analyte and a specific binding substance, (d) measuring, based on the measurement pattern, the signal intensity at least twice in a period in which a signal intensity attributed to the specific binding reaction reaches saturation, after the step (c); and (e) determining, based on the database, an amount of the analyte in the sample by using at least two signal intensities obtained in the step (d).

WO 91/04489 discloses a homogeneous immunoassay system for the determination of an antibody or an antigen in a sample which consists of an interferometric signal from an optical source, a waveguide coated with an antibody or an antigen and having at least one region immersed in a solution containing a sample, whereby the corresponding antigen or antibody can be complexed on the waveguide, a detector adapted to measure the interferometric signal after its propagation through the waveguide, and a measuring device to take the Fourier transform of the interferometric signal for determining the degree of attenuation of the interferometric signal at a wavelength corresponding to an absorption characteristic of the antigen-antibody complex or of a label incorporated into the antigen-antibody complex, whereby determining the amount of antigen or antibody in the sample.

### SUMMARY OF THE INVENTION

The present invention has been devised in view of the above-described situations. It is therefore an object of the present invention to provide a simple and highly accurate immunoassay method.

An immunoassay method according to the present invention is an immunoassay method for measuring a concentration of a substance to be measured and contained in a sample solution, the method including the steps of: a) mixing the sample solution and a specific binding substance capable of specifically binding to the substance to be measured and obtaining, with a predetermined sampling interval, a change with time in an optical change amount in a reaction of the substance to be measured and the specific binding substance as discrete measurement data; b) selecting a time interval of the measurement data including a time when an increase rate of the optical change amount is maximum; c) performing, with the time interval assumed to be a period of a periodic function, discrete Fourier transform to the measurement data within the time interval to obtain an amplitude spectrum distribution; and d) judging, based on a shape of the amplitude spectrum distribution, whether or not a zone phenomenon has occurred in the reaction in the step a). In the method, a combination of the substance to be measured and the specific binding substance is a combination of an antigen and an antibody.

According to the immunoassay method of the present invention, the content (concentration) of a substance to be measured can be obtained. Specifically, according to the present invention, the optical change amount of a reaction system can be measured while whether or not the zone phenomenon has occurred in the reaction system is judged. Therefore, a value for the content (concentration) of the substance to be measured obtained based on the optical change amount is highly reliable. Moreover, the optical change amount of the reaction system can be measured while whether or not the zone phenomenon has occurred in the reaction system is judged. Therefore, measurement procedures can be very simple. Furthermore, according to the present invention, Fourier transform is used. Thus, for a change in measurement data, noise can be considered to be a high frequency signal and a signal due to the change in measurement data can be separated from a signal due to noise. Accordingly, the immunoassay method of this embodiment is hardly influenced by noise.

The optical change amount may be an amount of a change in scattered light intensity or an amount of a change in transmitted light.

The time interval may have been determined beforehand based on measurement data for the optical change amount measured in a reaction in which no zone phenomenon occurs.

The time interval may be from a time when the specific binding substance and the sample solution are mixed to a time when the optical change amount substantially becomes constant after the reaction has been substantially saturated.

It is preferable that in the step d), whether or not the zone phenomenon has occurred in the reaction in the step a) is judged, based on the shape of the amplitude spectrum distribution and a shape of the amplitude spectrum distribution measured in a reaction in which no zone phenomenon occurs.

In the step e), whether or not the zone phenomenon has occurred in the reaction in the step d) may be judged, based on the shape of the amplitude spectrum distribution and a shape of the amplitude spectrum distribution measured in a reaction in which no zone phenomenon occurs.

It is preferable that in the step d), whether or not the zone phenomenon has occurred in the reaction in the step a) is judged, based on a ratio of an amplitude value at a frequency corresponding to a reciprocal of the time interval to a direct current component (i.e., an amplitude value at a frequency of 0) of the amplitude spectrum distribution.

It is preferable that in the step d), it is judged that no zone phenomenon has occurred in the reaction in the step a) if the ratio is higher than a reference value obtained from respective distributions of a reaction in which no zone phenomenon occurs and a reaction in which the zone phenomenon occurs, and the zone phenomenon has occurred in the reaction in the step a) if the ratio is lower than the reference value.

The immunoassay method of the present invention may further includes before the step a), the step e) of constructing a reaction system including only one of the sample solution and the specific bonding substance and obtaining, with a predetermined sampling interval, the change with time in the optical change amount in the reaction system as discrete measurement data. In the method, in the step a), the other one of the sample solution and the specific bonding substance which is not contained in the reaction system may be added to the reaction system to mix the sample solution and the specific binding substance, and the time interval may be from a time before the specific binding substance and the sample solution are mixed to a time when the optical change amount substantially becomes constant after the reaction has been substantially saturated.

It is preferable that in the step d), it is judged that no zone phenomenon has occurred in the reaction system in the step a) if a difference between an amplitude value at a frequency corresponding to a reciprocal of a time interval from a time when the specific binding substance and the sample solution are mixed to a time when a reaction in the reaction system is substantially saturated and an amplitude value at a frequency corresponding to a reciprocal of a time interval from the time when the specific binding substance and the sample solution are mixed to a time when the optical change amount substantially becomes constant after the reaction in the reaction system has been saturated is a positive number, and the zone phenomenon has occurred in the reaction system in the step a) if the difference is a negative number.

A concentration of the substance to be measured may be quantified, based on an amplitude value of a direct current component of the amplitude spectrum distribution in the step c).

Optionally an immunoassay system according to the present invention might include: measurement means for measuring an optical change amount of a reaction to be caused when a sample solution containing a substance to be measured and a specific binding substance capable of specifically binding to the substance to be measured are mixed; measurement data processing means, connected to the measurement means, for selecting a time interval of the measurement data obtained by the measurement means including a time when an increase rate of the optical change amount is maximum and performing, with the time interval assumed to be a period of a periodic function, discrete Fourier transform to the measurement data within the time interval to obtain an amplitude spectrum distribution; and judgment means, connected to the measurement data processing means, for judging, based on a shape of the amplitude spectrum distribution, whether or not a zone phenomenon has occurred in the reaction.

With the optional immunoassay system of the present invention, the optical change amount of a reaction system can be measured while whether or not the zone phenomenon has occurred in the reaction system is judged. Therefore, a value for the content (concentration) of the substance to be measured obtained based on the optical change amount is highly reliable. Moreover, the optical change amount of the reaction system can be measured while whether or not the zone phenomenon has occurred in the reaction system is judged. Accordingly, measurement procedures can be very simple. Furthermore, according to the present invention, Fourier transform is used. Thus, for a change in measurement data, noise can be considered to be a high frequency signal and a signal due to the change in measurement data can be separated from a signal due to noise. Therefore, the immunoassay method of this embodiment is hardly influenced by noise. That is, according to this embodiment, a simple and highly reliable immunological measurement can be performed.

The time interval may be from a time when the specific binding substance and the sample solution are mixed to a time when the optical change amount substantially becomes constant after the reaction has been substantially saturated.

The optional immunoassay system may have a configuration in which the judgment means has stored beforehand, as a reference shape, a shape of an amplitude spectrum distribution in the case where no zone phenomenon has occurred in the reaction and compares the reference shape to a shape of the amplitude spectrum distribution obtained by the measurement data processing means, thereby performing judgment.

The optional immunoassay system may have a configuration in which the judgment means performs judgment, based on a ratio of an amplitude value at a frequency corresponding to a reciprocal of the time interval to a direct current component of the amplitude spectrum distribution.

The optional immunoassay system may have a configuration in which the judgment means judges that no zone phenomenon has occurred in the reaction in the measurement means if the ratio is higher than a reference value obtained from respective distributions of a reaction in which no zone phenomenon occurs and areaction in which the zone phenomenon occurs, and the zone phenomenon has occurred in the reaction in the measurement means if the ratio is lower than the reference value.

The time interval may be from a time before the specific binding substance and the sample solution are mixed to a time when the optical change amount substantially becomes constant after the reaction has been substantially saturated. In this case, it is preferable that the judgment means judges that no zone phenomenon has occurred in the reaction if a difference between an amplitude value at a frequency corresponding to a reciprocal of a time interval from a time when the specific binding substance and the sample solution are mixed to a time when a reaction in the reaction system is substantially saturated and an amplitude value at a frequency corresponding to a reciprocal of a time interval from the time when the specific binding substance and the sample solution are mixed to a time when the optical change amount substantially becomes constant after the reaction in the reaction system has been saturated is a positive number, and the zone phenomenon has occurred in the reaction if the difference is a negative number.

Moreover, the optional immunoassay system may further include quantification means, connected to the measurement data processing means, for quantifying a concentration of the substance to be measured, based on the shape of the amplitude spectrum distribution.

The quantification means for quantifying the concentration of the substance to be measured may quantify the concentration of the substance to be measured, based on the amplitude value of the direct current component of the amplitude spectrum distribution.

### BRIEF DESCRIPTION OF THE DRAWINGS

FIG. **1** is a flowchart illustrating respective steps of an immunoassay method according to an embodiment of the present invention.
FIG. **2A** is a graph showing discrete measurement data for an optical change amount in the embodiment of the present invention; and FIG. **2B** is a graph showing an amplitude spectrum distribution in the embodiment of the present invention.
FIG. **3** is a schematic view illustrating the configuration of an immunoassay system according to the embodiment of the present invention.
FIG. **4** is a graph showing results of plotting of mean values of measurement values obtained between from 150 seconds when an immunoreaction is sufficiently saturated to 200 seconds, in measurements of human albumin solutions by immunonephelometry according to an example of the present invention.
FIG. **5** is a graph showing changes with time in scattered light intensity obtained in measurement of human albumin solutions in an example of the present invention.
FIG. **6** is a graph showing an amplitude spectrum distribution obtained by performing discrete Fourier transform to changes with time in scattered light intensity obtained in measurement of a human albumin solution having a human albumin concentration of 5 mg/dl.
FIG. **7** is a graph showing an amplitude spectrum distribution obtained by performing discrete Fourier transform to changes with time in scattered light intensity obtained in measurement of a human albumin solution having a human albumin concentration of 10 mg/dl.
FIG. **8** is a graph showing an amplitude spectrum distribution obtained by performing discrete Fourier transform to changes with time in scattered light intensity obtained in measurement of a human albumin solution having a human albumin concentration of 20 mg/dl.
FIG. **9** is a graph showing an amplitude spectrum distribution obtained by performing discrete Fourier transform to changes with time in scattered light intensity obtained in measurement of a human albumin solution having a human albumin concentration of 30 mg/dl.
FIG. **10** is a graph showing an amplitude spectrum distribution obtained by performing discrete Fourier transform to changes with time in scattered light intensity obtained in measurement of a human albumin solution having a human albumin concentration of 50 mg/dl.
FIG. **11** is a graph showing an amplitude spectrum distribution obtained by performing discrete Fourier transform to changes with time in scattered light intensity obtained in measurement of a human albumin solution having a human albumin concentration of 100 mg/dl.
FIG. **12** is a graph showing an amplitude spectrum distribution obtained by performing discrete Fourier transform to changes with time in scattered light intensity obtained in measurement of a human albumin solution having a human albumin concentration of 200 mg/dl.
FIG. **13** is a graph showing an amplitude spectrum distribution obtained by performing discrete Fourier transform to changes with time in scattered light intensity obtained in measurement of a human albumin solution having a human albumin concentration of 300 mg/dl.
FIG. **14** is a graph showing amplitude spectrum distributions obtained by performing discrete Fourier transform to respective changes with time in scattered light intensity obtained in measurements of human albumin solutions having human albumin concentrations of 5 mg/ld and 300 mg/dl.
FIG. **15** is a graph showing respective changes with time in scattered light intensities in measurements of human albumin solutions having human albumin concentrations of 5 mg/ld and 300 mg/dl.
FIG. **16** is a graph showing results obtained by adding random noise to the measurement data in time intervals of FIG. 15.
FIG. **17** is a graph showing results obtained by shifting the measurement data of FIG. **15** by 0.5 seconds to create a difference.
FIG. **18** is a graph showing results obtained by shifting the measurement data of FIG. **16** by 0.5 seconds to create a difference.
FIG. **19** is a graph showing an amplitude spectrum distribution obtained by performing discrete Fourier transform to the measurement data of FIG. **16** in the case where random noise has been added.
FIGS. **20A** and **20B** are schematic views illustrating a reaction of an antigen and an antibody in a reaction system used in a homogeneous immunoassay method.

### DESCRIPTION OF THE PREFERRED EMBODIMENTS

In an immunoassay method used for measuring the content of a substance to be measured in a sample, the substance to be measured is brought into reaction with a specific binding substance capable of specifically binding to the substance to be measured, the amount of change in optical characteristics during the reaction is measured and the amount of substance to be measured is calculated from measurement results.

### (First immunoassay method)

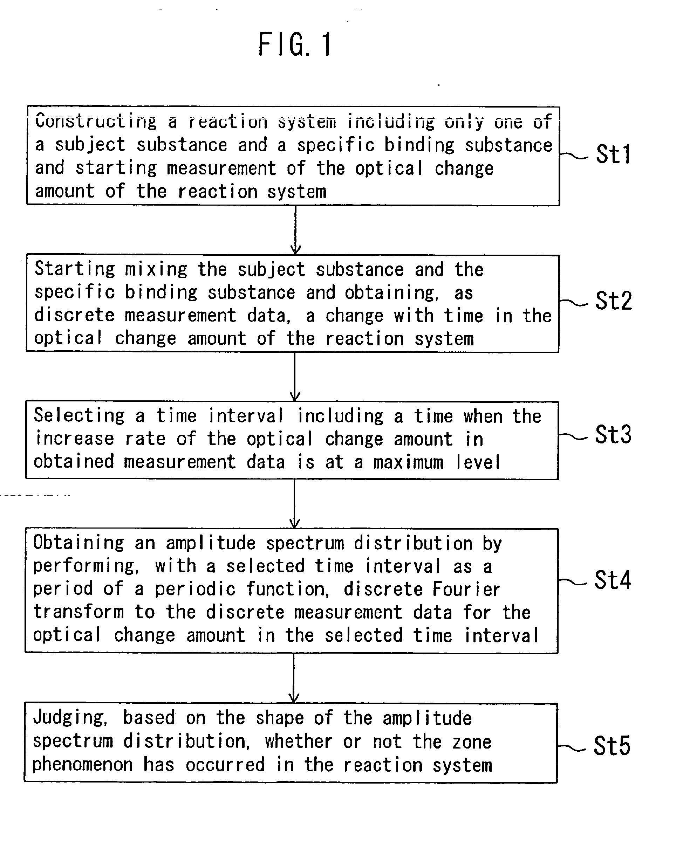

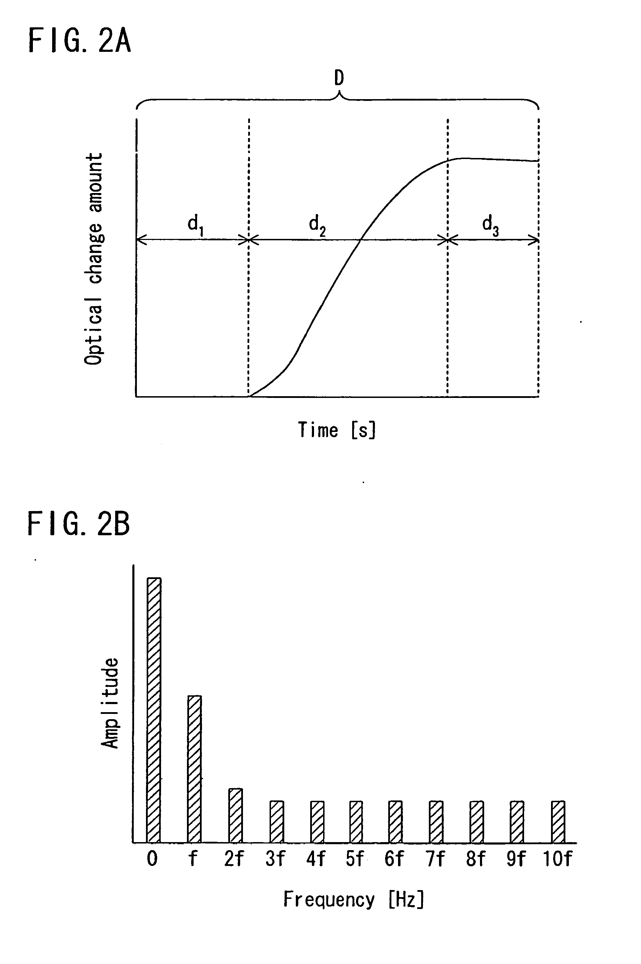

Hereinafter, a first immunoassay method according to the present invention will be described with reference to FIGS. **1** and **2**. FIG. **1** is a flowchart illustrating respective steps of the first immunoassay method of this embodiment. FIG. **2A** is a graph showing discrete measurement data for an optical change amount. FIG. **2B** is a graph showing an amplitude spectrum distribution. In FIG. **2B**, the reciprocal of a time interval (a period) discrete Fourier transformed is represented by **f** as a fundamental frequency. Note that the combination of the substance to be measured and the specific binding substance is the combination of antigen and antibody.

In the first immunoassay method of the present invention, first, in Step **St1** of FIG. **1** (which corresponds to the step e) in the claims) a reaction system including only one of the substance to be measured and the specific binding substance is constructed and measurement of the optical change amount of the reaction system is started. In this case, the reason why the reaction system includes only one of the substance to be measured and the specific binding substance is that the specific binding substance may be added to the substance to be measured later or the substance to be measured may be added to the specific binding substance later.

Next, in Step **St2** (which corresponds to the step a) in the claims), mixing of the substance to be measured and the specific binding substance is started and changes with time in the optical change amount of the reaction system are obtained as discrete measurement data. Note that the "optical change amount" is specifically the intensity of scattered light or the amount of change in transmitted light and the measurement of the optical change amount is performed with a constant sampling period. In this case, when the reaction system includes the substance to be measured, the specific binding substance is added to the reaction system, and when the reaction system includes the specific binding substance, the substance to be measured is added to the reaction system.

By Steps **St1** and **St2** described above, for example, measurement data for the optical change amount shown in FIG. **2A** is obtained. Note that the measurement data for the optical change amount of FIG. **2A** is indicated by a line joining discrete measurement results obtained at measurement times.

In the immunoassay method of this embodiment, when the substance to be measured is an antigen, the specific binding substance is antibody, and when the Substance to be measured is an antibody, the specific binding substance is an antigen. In Step **St1,** at the time when the reaction system includes only one of the substance to be measured and the specific binding substance, an agglutinate complex resulting from an antigen-antibody reaction of the substance to be measured and the specific binding substance is not generated in the reaction system. In contrast, in Step **St2**, when the substance to be measured and the specific binding substance are mixed, an agglutinate complex resulting from an antigen-antibody reaction of the substance to be measured and the specific binding substance starts being generated in the reaction system.

When agglutinates complexes are generated in the reaction system in Step **St2**, turbidity occurs in the reaction system, so that the optical change amount such as the intensity of scattered light and the amount of transmitted light varies. Therefore, by measuring the optical change amount, the degree of turbidity of the reaction system can be estimated. Note that as a reference for the estimate, the optical change amount when the reaction system includes only one of the substance to be measured and the specific binding substance, i.e., the amount of change in the scattered light intensity or the amount of change in the transmitted light amount is preferably measured.

Next, in Step **St3** of FIG. **1** (which corresponds to the step b) in the claims), in obtained measurement data, a time interval including a time at which the increase rate of the optical change amount (i.e., reaction rate) is at a maximum level is selected. Specifically, in FIG. **2A,** a time interval **d**_{**2**} + **d**_{**3**} are selected.

Next, in Step **St4** of FIG. **1** (which corresponds to the step c) in the claims), with the selected time interval **d**_{**2**} + **d**_{**3**} assumed to be a period of a periodic function, discrete Fourier transform is performed to discrete measurement data for the optical change amount in the selected time interval **d**_{**2**} + **d**_{**3**}**,** thereby obtaining, for example, the amplitude spectrum distribution of FIG. **2B.**

Next, in Step **St5** of FIG. **1** (which corresponds to the step d) in the claims), whether or not a zone phenomenon has occurred in the reaction system in Step **St2** is judged, based on the shape of the amplitude spectrum distribution. Specifically, in this case, the shape of the amplitude spectrum distribution in the case where the zone phenomenon has not occurred in the reaction system is obtained beforehand as a "reference shape", and then judgment is performed by comparison of the amplitude spectrum distribution obtained in Step **St4** to the reference shape. The judgment method will be described in detail later in Example 4. Note that in this step, the shape of the amplitude spectrum distribution can be judged by naked eyes or using computer. Moreover, as described later in Example 2, the concentration of the substance to be measured can be measured, based on an amplitude value of a direct current component in the amplitude spectrum distribution obtained in Step **St4**.

By performing the above-described steps, the content (concentration) of the substance to be measured can be obtained. Specifically, according to the immunoassay method of this embodiment, the optical change amount of the reaction system can be measured while whether or not the zone phenomenon has occurred in the reaction system is judged. Therefore, a value for the content (concentration) of the substance to be measured obtained based on the optical change amount is highly reliable.

Moreover, according to the immunoassay method of this embodiment, the optical change amount of the reaction system can be measured while whether or not the zone phenomenon has occurred in the reaction system is judged. Accordingly, it is not necessary to perform a plurality of measurements and also to prepare a plurality of reaction vessels for each sample solution. Therefore, measurement procedures can be very simple.

Furthermore, in the immunoassay method of this embodiment, Fourier transform is used. Thus, for a change in measurement data, noise can be considered to be a high frequency signal and a signal due to the change in measurement data can be separated from a signal due to noise. Accordingly, the immunoassay method of this embodiment is hardly influenced by noise. Therefore, according to this embodiment, a simple and highly reliable immunoassay method can be provided.

Note that in the immunoassay method of this embodiment, it is preferable that the optical change amount is the amount of change in the scattered light intensity or the amount of change in the transmitted light amount. This is because immunoturbidimetry for measuring a change in the transmitted light amount and immunonephelometry for measuring a change in the amount of scattered light, which are dependant on the generation of an antigen-antibody agglutinate complex, are apt to be influenced by the zone phenomenon and therefore great effects can be achieved with use of the assay technique of this embodiment.

Moreover, it is preferable that each of the time intervals **d2** and **d3** selected in Step **St3** is a time interval determined based on measurement data for the optical change amount in the case where the zone phenomenon has not occurred. Specifically, it is preferable that a time interval obtained by not measuring a reaction time of the reaction system to be measured but measuring beforehand a reaction time in the case where no zone phenomenon occurs is used as for each of **d2** and **d3**. This is because measurement data in the case where the zone phenomenon has not occurred exhibits clear information such as a time at which the increase rate (reaction rate) of the optical change amount of the reaction system reaches its peak and a time at which an antigen-antibody reaction is saturated, compared to measurement data in the case where the zone phenomenon has occurred, and is highly stabile, so that selection of a reference position can be made in a simple manner in time interval selection.

Specifically, the time interval **d**_{**2**} + **d**_{**3**} for the measurement data selected based on the measurement date for the optical change amount in the case where the zone phenomenon has not occurred is preferably a time interval from a time when the specific binding substance and a sample solution is mixed (i.e., a time when a reaction is started) to a time when after the reaction has been substantially saturated, the optical change amount of the reaction system becomes substantially constant. Thus, a discrete Fourier transformed spectrum which reflects a difference between the measurement data in the case where the zone phenomenon has not occurred and the measurement data in the case where the zone phenomenon has occurred can be obtained, so that whether or not the zone phenomenon has occurred in the reaction system in Step **St2** can be more clearly judged.

Note that in this specification and the claims, the optical change amount in the phrase "the optical change amount (of the reaction system) becomes substantially constant" is meant to be the optical change amount in the time interval **d**_{**3**} of FIG. **2A** and the phrase "the optical change amount (of the reaction system) becomes substantially constant" includes not only the case where a measured optical change amount is constant but also the case where the optical change amount is changed by an amount with which the change can be clearly recognized, compared to the optical change amount in the time interval **d**_{**2**} of FIG. **2A.** Moreover, in this specification and the claims, a time when "the reaction is substantially saturated" is specifically meant to be a boundary time between the time interval **d**_{**2**} and the time interval **d**_{**3**} in FIG. **2A** and also part in which the largest change in the inclination of the graph indicating the optical change amount is observed.

Moreover, in Step **St5**, in the discrete Fourier transformed amplitude spectrum distribution been and obtained in Step **St4**, whether or not an antigen antibody reaction has occurred in a zone region is preferably judged, based on change in the amplitude value of a direct current component with respect to the frequency. Amplitude values in the amplitude spectrum distribution markedly reflect differences in rises of a reaction curve representing an immunological reaction. Thus, whether or not the zone phenomenon has occurred in the reaction system in Step **St2** can be judged in a simple manner.

Moreover, the concentration of the substance to be measured is preferably measured, based on the amplitude value of a direct current component in the amplitude spectrum distribution obtained in Step **St4**. Thus, influence of noise on measurement data can be reduced and also discrete Fourier transformed data can be effectively used.

### (Second immunoassay method)

Next, unlike in the first immunoassay method, the case where the time interval from the time when a reaction is Started to the time when after the reaction has been substantially saturated, the optical change amount becomes substantially constant (i.e., the time interval **d**_{**2**} + **d**_{**3**} shown in FIG. **2A**) is not selected but a time interval from the time before the specific binding substance and the sample solution are mixed (i.e., a time before a reaction is started) to the time when after the reaction has been substantially saturated, the optical change amount becomes substantially constant is selected as a time interval will be described.

In this case, the same measurement as that in the first immunoassay method is performed in Steps **St1** and **St2** of FIG. **1,** the time interval **d**_{**1**} + **d**_{**2**} + **d**_{**3**} is selected in Step **St3**, and, discrete Fourier transformation is performed, with the time interval **d**_{**1**} + **d**_{**2**} + **d**_{**3**} assumed to be a period, to obtain an amplitude spectrum distribution. Then, in Step **St5**, based on a value obtained by subtracting an amplitude value at a frequency (1/(d₂ + d₃)) corresponding to the reciprocal of a sum (d₂ + d₃) of the time interval (**d**_{**2**}) from the time when an antigen-antibody reaction is started and the time when the reaction is substantially saturated and the time interval (d₃) from the time when the reaction is substantially saturated to the time when the optical change amount becomes substantially constant from an amplitude value at a frequency (1/d₂) corresponding to the reciprocal of the time interval from the time when a reaction is started to the time when the reaction reaches its saturation (i.e., the time interval **d**_{**2**} of FIG. **2**), whether or not the antigen-antibody reaction has occurred in the zone region can be judged. In this case, if a difference of {(an amplitude value of 1/d₂) - (an amplitude of 1/(d₂ + d₃))} is a positive number, it is judged that no zone phenomenon has occurred in the reaction system, and if it the difference is a negative number, it is judged that the zone phenomenon has occurred in the reaction system. Thus, whether or not the zone phenomenon has occurred in the reaction system can be judged in a simple manner.

In this case, assume that the time interval from the time before the reaction is started to the time when the reaction is saturated is selected. If selection of a time interval is made so that each of the time interval **d**_{**1**} indicating a time interval before a reaction, the time interval **d**_{**2**} indicating a time interval in which the reaction rate is being increased, and the time interval **d**_{**3**} indicating a time interval after the reaction has been saturated is defined to have the same length, a value for the difference can be more clearly shown.

### (Immunoassay system)

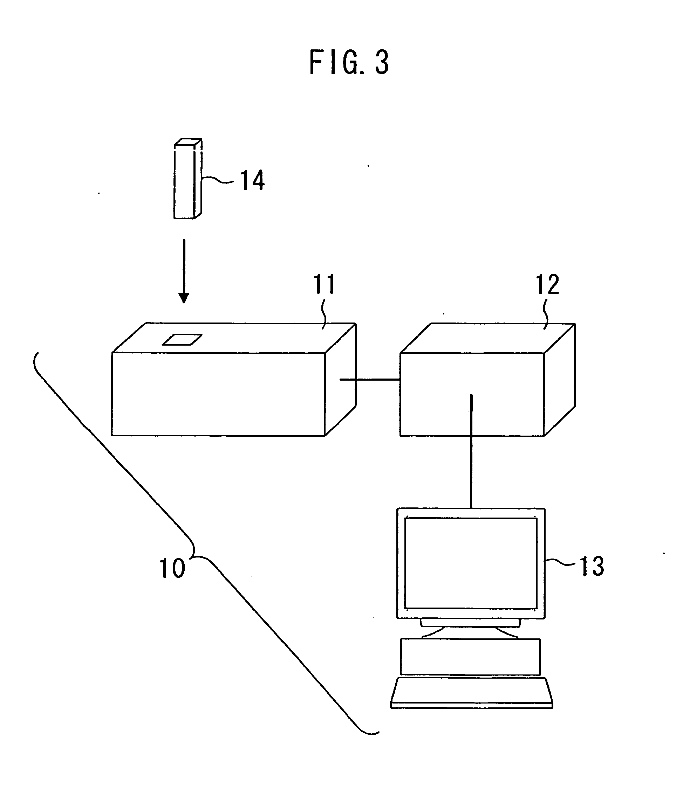

Next, an optional immunoassay system used in the above-described immunoassay method will be described with reference to FIG. **3**. As shown in FIG. **3**, an immunoassay system **10** according to this embodiment includes measurement means **11**, measurement data processing means **12** electrically connected to the measurement means **11**, and judgment/quantification means **13** electrically connected to the measurement data processing means **12**. Note that in this specification, "connection" or "to connect" means "electrically connection" or "to electrically connect" unless otherwise specified.

The measurement means **11** is for accommodating a measurement cell **14** and measuring the optical change amount of a reaction system constructed in the measurement cell **14**. For example, as the measurement means **11**, a system which allows measurement of the amount of change in the scattered light intensity or the amount of change in the transmitted light amount as the optical change amount is used. Note that in this embodiment, the measurement means **11** is made to have a configuration in which the measurement cell **14** is introduced into the measurement means **11** when measurement is performed. However, the configuration of the measurement means **11** is not limited thereto, but may be a configuration in which a measurement cell is arranged in the measurement means **11**.

The measurement data processing means **12** selects, in measurement data obtained by the measurement means **11,** a time interval (e.g., the time interval **d**_{**2**} + **d**_{**3**} or the time interval of **d**_{**1**} + **d**_{**2**} + **d**_{**3**} in FIG. **2A**) including a time at which the increase rate of the optical change amount (i.e., reaction rate) is at a maximum level and performs, with a selected time interval **D** assumed to be a period of a periodic function, discrete Fourier transformation to discrete measurement data for the optical change amount in the selected time interval **d**_{**2**} + **d**_{**3**} or **d**_{**1**} + **d**_{**2**} + **d**_{**3**}**.** Thus, for example, the amplitude spectrum distribution of FIG. **2B** is obtained.

The judgment/quantification means **13** judges, based on the shape of the amplitude spectrum distribution obtained by the measurement data processing means **12**, whether or not the zone phenomenon has occurred in the reaction system in the measurement cell **14** and quantifies, if it is judged that the zone phenomenon has not occurred, the concentration of the substance to be measured. Note that the judgment/quantification means **13** stores beforehand as "reference shape" the shape of the amplitude spectrum distribution in the case where the zone phenomenon has not occurred in the reaction system in a state of electric data beforehand and then compares an amplitude spectrum distribution obtained by the measurement data processing means **12** to the reference shape, thereby performing judgment. Moreover, the judgment/quantification means **13** stores beforehand a mathematical expression (calibration curve) in which amplitude values of specific frequency components are matched off against concentrations of the substance to be measured in a state of electric data in order to perform quantification. The amplitude value of a specific frequency component obtained by the measurement data processing means **12** is substituted into the mathematical expression (calibration curve), thereby obtaining the concentration of the substance to be measured.

The measurement cell **14** is formed of a transparent material and can retain a fluid inside. When measurement is performed, a reaction system is injected into the cell and then the cell is introduced into the measurement means **11**. A specific binding substrate and a sample solution are mixed while measurement of the optical change amount continues to be performed with a constant sampling period.

Next, the operation of the immunoassay system **10** of this optional embodiment will be described.

First, a substance to be measured or a specific binding substance is introduced into the measurement cell **14** to construct a reaction system, and then the measurement cell **14** is accommodated in the measurement means **11.**

Next, in the measurement means **11**, measurement of the optical change amount of the reaction system is started. In this case, measurement of the optical change amount of the reaction system is performed with a constant sampling period.

Subsequently, the specific binding substance capable of specifically binding to the substance to be measured is mixed with a sample solution while measurement of the optical change amount of the reaction system continues to be performed with the above-described sampling period. Then, changes with time in the optical change amount of the reaction system are obtained as discrete measurement data for the optical change amount (e.g., the measurement data for the optical change amount shown in FIG. **2A**).

Note that the measurement means **11** is preferably formed to have a configuration in which using, as a reference, the reaction system including only one of the substance to be measured and the specific binding substance, the optical change amount of the reaction system (e.g., the amount of change in the scattered light intensity or the amount of change in the transmitted light amount) is measured.

Next, the measurement data processing means **12** selects, in measurement data obtained by the measurement means **11**, a time interval including a time at which the increase rate of the optical change amount (i.e., reaction rate). Specifically, in FIG. **2A**, the time interval **d**_{**2**} + **d**_{**3**} or the time interval **d**_{**1**} + **d**_{**2**} + **d**_{**3**} are selected.

Next, the measurement data processing means **12** assumes the time interval **d**_{**2**} + **d**_{**3**} or the time interval **d**_{**1**} + **d**_{**2**} + **d**_{**3**}**,** which has been selected, as a period of a periodic function and performs discrete Fourier transform to discrete measurement data for the optical change amount in the selected time interval **d**_{**2**} + **d**_{**3**} or the time interval **d**_{**1**} + **d**_{**2**} + **d**_{**3**}**,** for example, thereby obtaining the amplitude spectrum distribution of FIG. **2B.**

Next, the judgment/quantification means **13** judges, based on the shape of the amplitude spectrum distribution obtained by the measurement data processing means **12**, whether or not the zone phenomenon has occurred in the reaction system in the measurement cell **14** and, moreover, the concentration of the substance to be measured is quantified.

With the immunoassay system **10** of this optional embodiment, the optical change amount of a reaction system can be measured while whether or not the zone phenomenon has occurred in the reaction system is judged. Accordingly, reliability of a numerical value for the content (concentration) of a substance to be measured obtained based on the optical change amount is very high. Therefore, according to this embodiment, a simple and highly reliable immunological measurement can be performed.

Note that an absorptiometer is a specific example of the measurement means **11**. Moreover, the measurement data processing means **12** is preferably formed to have a configuration in which measurement data is obtained from the measurement means **11** through A/D conversion using an A/D conversion device.

The immunoassay system **10** of this optional embodiment is formed to have a configuration in which the measurement data processing means **12** and the judgment/quantification means **13** are separately provided. However, the immunoassay system **10** is not limited thereto. For example, the measurement data means **12** and the judgment/quantification means **13** may be formed using software and a computer. Moreover, the measurement data means **12** and the judgment/quantification means **13** may be formed using a circuit element such as DSP (digital signal processor).

When a computer used as the measurement data processing means **12**, a configuration in which selection reference and range of a time interval in the measurement data processing means **12** are arbitrarily specified by a user with software may be adopted.

Moreover, when the measurement data processing means **12** is a computer and measurement is performed using a specific sample solution, a configuration in which a time interval to be selected by the measurement data processing means **12** can be stored on a memory such as a ROM and RAM is preferably used. In this case, the time interval to be stored is preferably a time interval based on the measurement data of the optical change amount in the case where the zone phenomenon has not occurred.

When a computer is used as the judgment/quantification means **13**, a configuration in which a reference shape used for judgment of the zone phenomenon is arbitrarily set by a user with software may be used.

Moreover, when the judgment/quantification means **13** is a computer and measurement is performed using a specific sample solution, a configuration in which the reference shape can be stored on a memory such as a ROM and a RAM is preferably adopted. Furthermore, the reference shape is preferably an amplitude spectrum distribution including respective amplitude values at different frequencies.

As has been described, with the immunoassay method (of this embodiment, it is not necessary to perform a plurality of measurements and to prepare a plurality of reaction vessels for each sample solution. Moreover, long wavelength measurement is not necessary. Accordingly, measurement procedures and a system configuration become very simple.

Specifically, in the immunoassay method of this embodiment, whether or not the zone phenomenon is caused can be judged by measurement using light having a wavelength. Accordingly, a system configuration becomes simple and measurement process steps do not become complicated. Moreover, Fourier transform is used, so that for a change in measurement data, noise can be considered as a high frequency signal and a signal due to a change in measurement data and a signal due to noise can be separated. Therefore, the immunoassay of this embodiment are hardly influenced by noise.

### (Examples)

Hereinafter, the present invention will be described using examples. However, the present invention is not limited to the examples.

### - Example 1-

Hereinafter, an example of the specific configuration of the immunoassay system 10 shown in the above-described embodiment will be described. Note that in this example, description will be made using the second immunoassay method (in which the time interval **d**_{**1**} + **d**_{**2**} + **d**_{**3**} is selected) of the first and second immunoassay methods described above.

In this example, the measurement means **11** is so configured to have the following configuration. A semiconductor laser pointer (MLXS-D-12-680-35 available from Kiko Giken) for outputting at an output power of 15 mW laser light modulated at 270 Hz and having a wavelength of 680 nm and is used as a light source and a silicon photodiode for a visible to infrared radiation precision photometry (S2387-66R available from Hamamatsu Photonics) is used as a detector. As a measurement cell, a measurement cell formed of 0.1 cm thick optical glass plates glued to one another and having a regular quadrangular prism shape and a capacity of about 200 µl is used. As for the arrangement of each of the light source, the detector and the measurement cell, the measurement cell is located at a 0.5 cm distance from the light source so that a surface of the measurement cell is vertical to the optical axis of incident light from the light source, and the detector is located at a 5.5 cm distance from the measurement cell in the direction in which the detector and the light source make an angle of 90 degree with respect to the measurement cell. Between the detector and the cell, a shading cylinder is provided so that stray light does not enter the detector. An electric current signal depending on the quantity of light detected by the detector is amplified to be a 100 times large voltage signal through a current-voltage conversion circuit (10⁶ V/A) and an amplification circuit using an operational amplifier, phase sensitive detected via a lock-in amplifier (5610B available from NF corporation) and then is loaded into a computer at regular intervals by GPIB control. In this example, a sampling period is set at 0.5 seconds.

The measurement processing means **12** is formed to have the following configuration. In this example, a time interval to be selected is set so that the time interval is within a range from the time before a reaction is started to the time when a certain time has lapsed after the reaction has been saturated and so that a data length before the reaction is started, during the reaction is increased and a data length after the reaction has been saturated are substantially the same. For the antigen-antibody reaction later described in Example 2, sampling data was obtained from 64 points in a time interval from 10 seconds before a start of a reaction to 22 seconds after the start of the reaction. The obtained data was then assumed to be a periodic signal with a period of 32 seconds and a frequency of 0.03125 Hz and is discrete Fourier transformed on Microsoft Excel (available from Microsoft), thereby obtaining an amplitude spectrum distribution.

As judgment means in the judgment/quantification means **13**, adopted was a method in which a substance to be measured and a specific binding substance are mixed and an amplitude value at a frequency corresponding to the reciprocal (1/d₂) of the time interval (**d**_{**2**} in FIG. **2A**) from the time when a reaction is started to the time when the reaction reaches its saturation is compared to an amplitude value corresponding to the reciprocal (1/ (d₂+ d₃)) the sum of the time interval (**d**_{**2**} in FIG. **2A**) from the time when a reaction is started to the time when the reaction becomes saturated and the time interval (**d**_{**3**} in FIG. **2A**) from the time when the reaction is substantially saturated to the time when the optical change amount substantially becomes constant. Specifically, a value of {(an amplitude at 0.0938 Hz) - (an amplitude at 0.0625 Hz)}, i.e., a difference between an amplitude at a frequency of 0.0938 Hz corresponding to the reciprocal of an about 10 second interval (i.e., **d**_{**2**}) and an amplitude at a frequency of 0.0625 Hz corresponding to the reciprocal of an about 15 second interval (i.e., d₂ + d₃) is obtained by calculation to judge that the zone phenomenon has not occurred if the value is a positive number and the zone phenomenon has occurred if the value is a negative number.

Moreover, as quantification means, a method in which quantification is performed using an amplitude value of a direct current component in an amplitude spectrum distribution is adopted. Specifically, the amplitude value of the direct current obtained by the measurement of the substance to be measured is substituted into a mathematical expression (calibration curve) in which an amplitude value of a direct current component is matched off against the concentration of the substance to be measured, thereby obtaining the concentration of the substance to be measured.

### (Example 2)

In this example, it will be shown that the judgment described in the first example is valid. In this case, an antigen-antibody reaction is caused using a substance to be measured, of which the concentration is known, and an analysis is conducted to confirm which concentration the zone phenomenon occurs at. Then, comparison is made between amplitude spectra in the case where the zone phenomenon has occurs and the case where the zone phenomenon has not occurred. In this case, an example of an immunoassay method in which the substance to be measured is human albumin will be described. Note that pure water filtered by Milli-Q SP TOC (available from Millipore) is used for preparing a buffer solution described below or the like. Moreover, note that as reagents such as salt and buffering agents, those from Wako Pure Chemical Industries, Ltd. were used unless otherwise specified, and a reagent of first grade is used for polyethylene glycol 6000, while the extra pure grade was used for the others.

A rabbit antihuman albumin polyclonal antibody (which will be hereinafter referred to as an "antibody") solution was obtained by purifying an antiserum collected from a rabbit immunized against human albumin by protein A column chromatography and then dialyzing out the purified antiserum. The column was filled with protein A stationary gel from Amersham Pharmacia. An equilibrium buffer solution composed of 1.5 M glycine and 3.0 M NaCl, pH 8.9 was used for purification, and an elution buffer solution composed of a citric acid solution (0.1 M citric acid, pH 4.0) was used. The eluted antibody fraction was put in a dialysis tube having a fractional molecular weight of 10,000, and dialyzed several times with about a hundred-fold volume of a buffer solution composed of 0.05 M 3-(N-morpholino) propanesulfonic acid (which is available from Dojin and will be hereinafter referred to as "MOPS"), 0.15 M NaCl and 0.04 wt.% NaN₃, pH 7.4, for replacement of buffer components. Thereafter, the antibody concentration was estimated by 280 nm absorbance measurement, and adjusted with the same buffer solution as that used for the dialysis to obtain an antibody concentration of 3.0 mg/ml.

Human albumin solutions were prepared so as to have human albumin concentrations of 0, 5, 10, 20, 30, 50, 100, 200, and 300 mg/dl by dissolution using a human albumin (available from Wako Pure Chemical Industries, Ltd.) solution (i.e., a sample solution) as the antigen and with the same buffer solution as that used for the dialysis. Buffer solutions having a composition of 0.05 M MOPS and 4 wt.% polyethylene glycol 6,000, pH 7.4 were used for measurement.

For each buffer solution, the measurements of human albumin solutions having respective concentrations were performed in the following manner. Each reaction system has a composition of 160 µl of the buffer solution, 9 µl of the human albumin solution and 23 µl of the antibody solution. Therefore, the final concentration of the antibody in the reaction system will be about 0.11 mg/ml, and the final concentration of the human albumin will be a value obtained by multiplying the concentration of the human albumin solution used for the measurement by 0.046.

First, the buffer solution and the human albumin solution having respective volmes described above were mixed by stirring in the cell of the immunoassay system configured in the above-described manner so as to prepare a reaction system. Subsequently, the antibody solution having the above described volume was added to the reaction system and stirred, to cause an antigen-antibody reaction. Measurement of scattered light was started 10 seconds before the addition of the antibody solution and performed at intervals of 0.5 seconds for 200 seconds. The measurement values were obtained as voltage values. To prevent influence of contamination of the measurement cell on the measurement, correction was made with a value measured by putting pure water in the measurement cell before measurement of each reaction. The measurement was performed at room temperature (about 20°C).

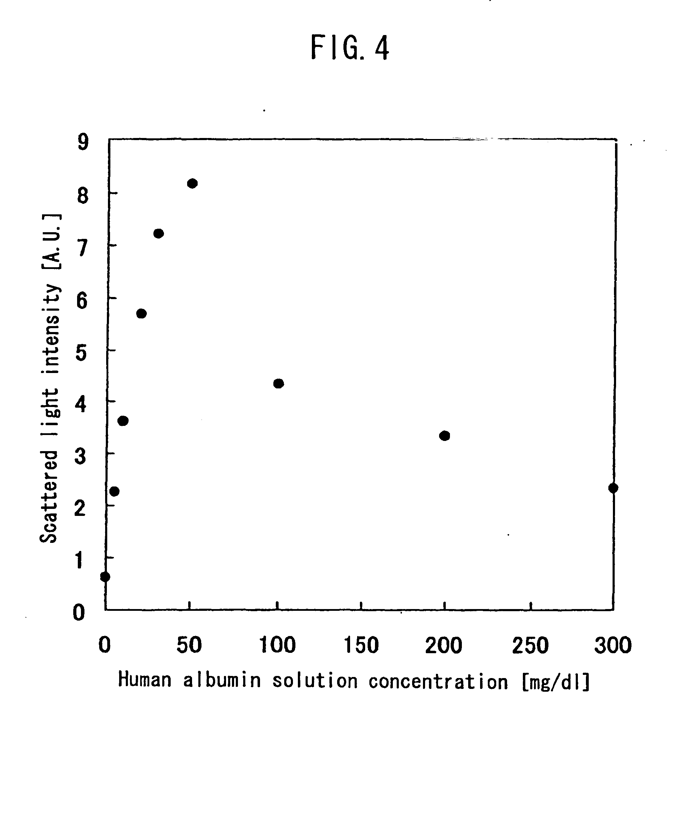

FIG. **4** is a graph showing results of plotting of mean values of measurement values between from 150 seconds when an immunoreaction has been sufficiently saturated to 200 seconds. From FIG. **4**, it can be seen that the measurements of human albumin solutions having a human albumin concentration of 100 mg/dl or more is influenced by the zone phenomenon.

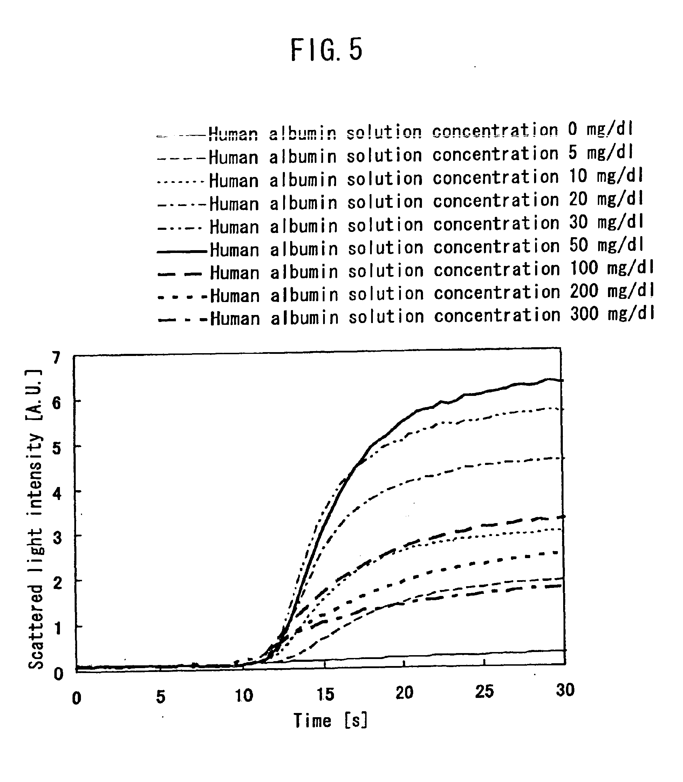

FIG. **5** is a graph showing changes with time in scattered light intensity obtained in measurement of each human albumin solution. Discrete Fourier transform for detecting the zone phenomenon was performed to measurement values obtained in the measurement.

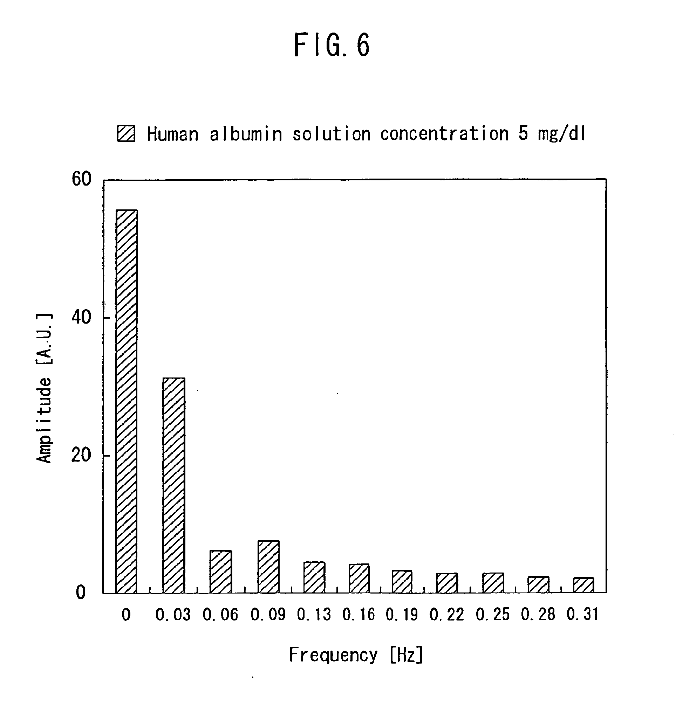

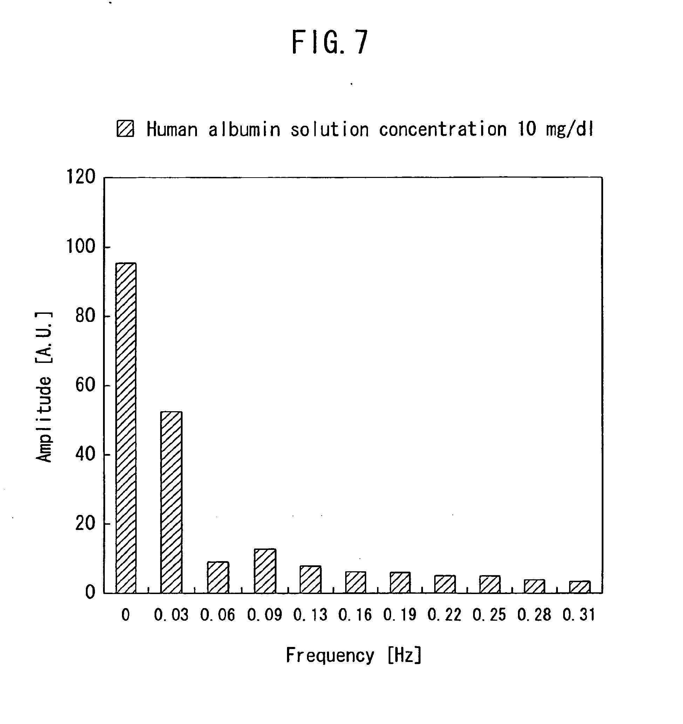

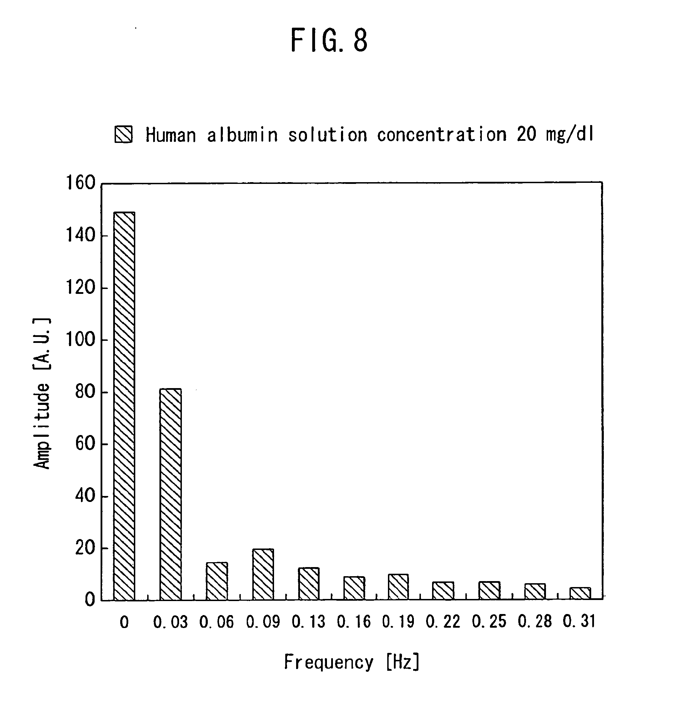

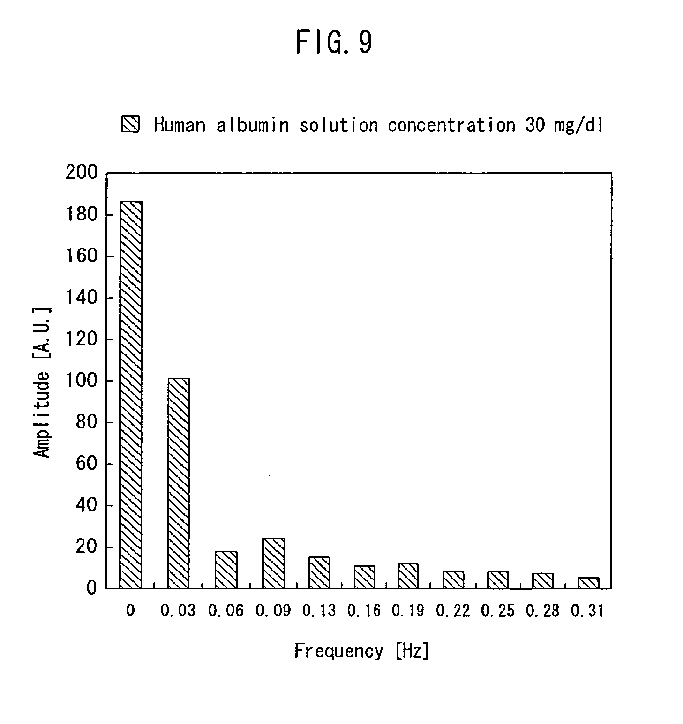

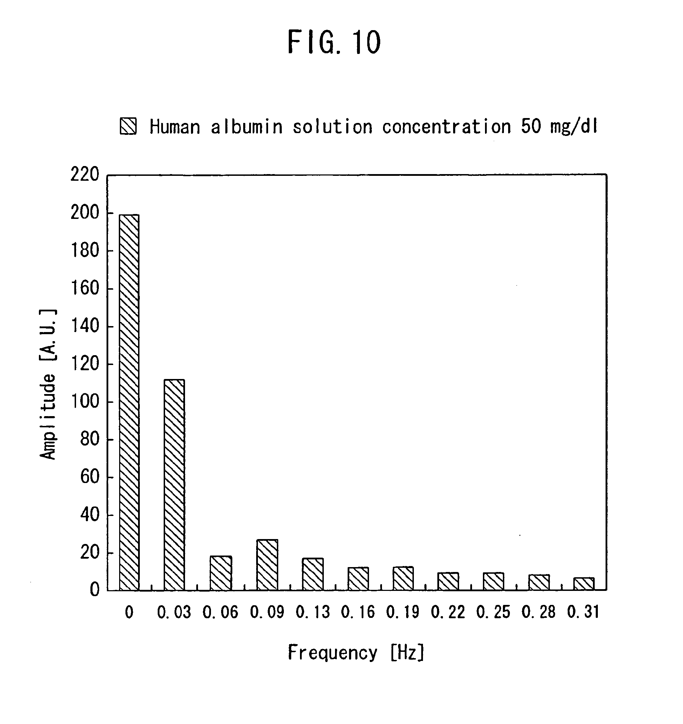

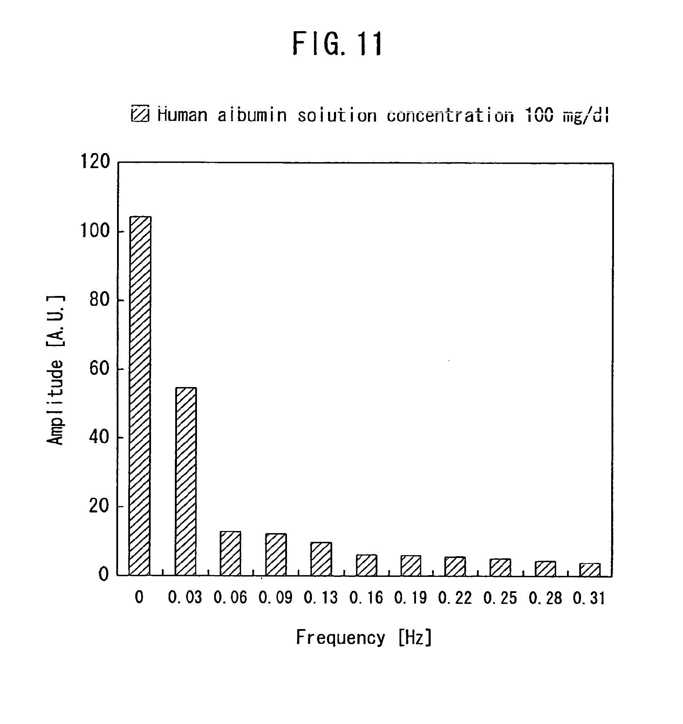

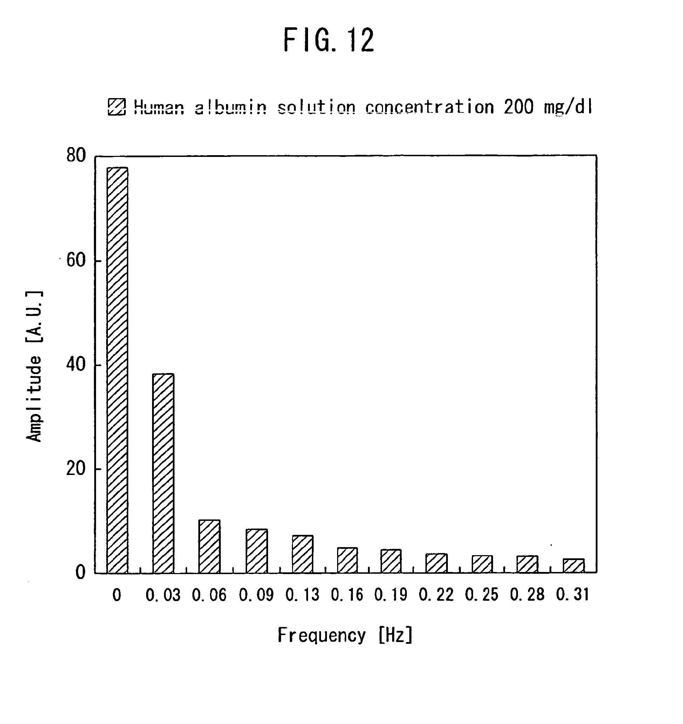

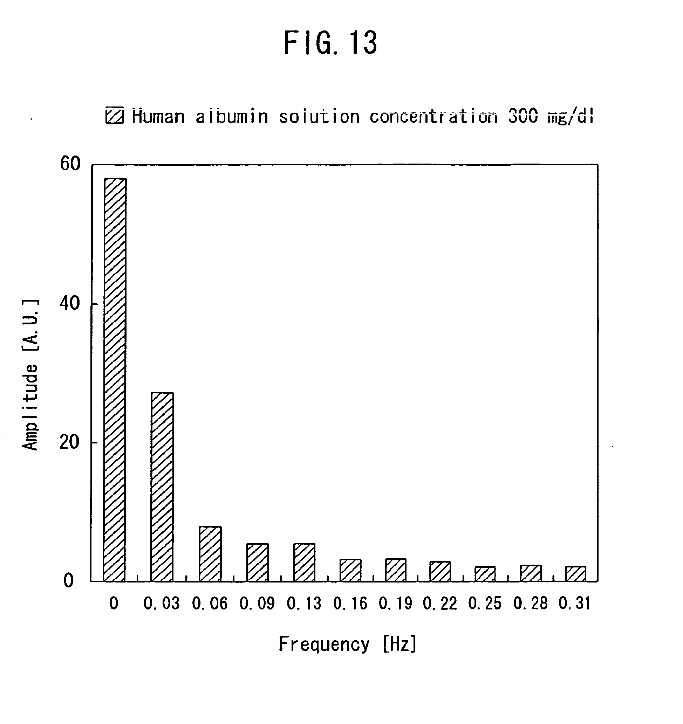

FIGS. **6** through **13** are graphs showing amplitude spectrum distributions obtained from performing discrete Fourier transform to measurement data obtained from measurements of the human albumin solutions having respective human albumin concentrations of 5 mg/dl through 300 mg/dl. According to the resultant distributions, in each of human albumin solutions having concentrations of 5 mg/dl through 50 mg/dl in which the zone phenomenon has not occurred, it can be confirmed that a value of {(an amplitude value at 0.0938 Hz) - (an amplitude at 0.0625 Hz)} is positive. Moreover, in each of the human albumin solutions having respective human albumin concentrations of 100 mg/dl through 300 mg/dl in which the zone phenomenon has occurred, it can be confirmed that a value of {(an amplitude value at 0.0938 Hz) - (an amplitude at 0.0625 Hz)} is negative. Actual calculation values were 1.65 for the human albumin solution having a human albumin concentration of 5 mg/dl, 3.37 for the human albumin solution having a human albumin concentration of 10 mg/dl, 5.54 for the human albumin solution having a human albumin concentration of 20 mg/dl, 5.80 for the human albumin solution having a human albumin concentration of 30 mg/dl, 8.69 for the human albumin solution having a human albumin concentration of 50 mg/dl, -0.962 for the human albumin solution having a human albumin concentration of 100 mg/dl, -1.93 for the human albumin solution having a human albumin concentration of 200 mg/dl, and 2.44 for the human albumin solution having a human albumin concentration of 300 mg/dl.

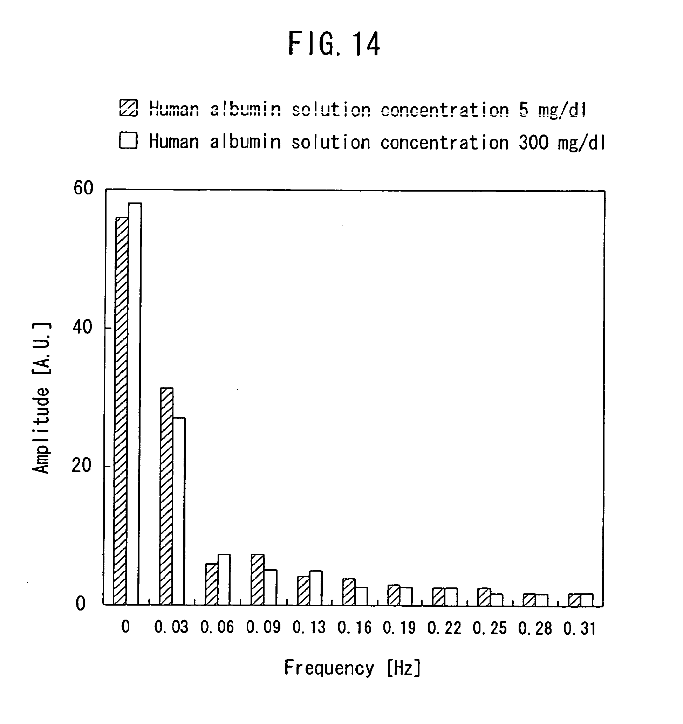

FIG. **14** is a graph showing comparison between the amplitude spectrum distribution of the human albumin solution having a human albumin concentration of 5 mg/dl and the amplitude spectrum distribution of the human albumin solution having a human albumin concentration of 300 mg/dl, which exhibit substantially the same changes with time as that shown FIG. **5**. As can be seen from FIG. **14**, the respective amplitude values at 0.0938 Hz and at 0.0628 Hz are inversed between the amplitude spectrum distribution of the human albumin solution having a human albumin concentration of 5 mg/dl in which the zone phenomenon does not occur and the human albumin solution having a human albumin concentration of 300 mg/dl in which the zone phenomenon occurs. That is, which positive or negative the value of {(an amplitude value at 0.0938 Hz) - (an amplitude at 0.0625 Hz)} is reflects whether or not the zone phenomenon has occurred is reflected on.

Subsequently, concentration quantification results will be described. A calibration curve was made using quantization results for human albumin solutions having human albumin concentrations of 5, 10, and 20 mg/dl obtained above. The obtained calibration curve was expressed by y = 6.12x + 29.1 where the amplitude value of a direct current component was y and the concentration of the human albumin solution was **x**. To prove that this calibration curve was valid, results from measurement of the amplitude value of a direct current component for the human albumin solution having a human albumin concentration of 15 mg/dl were substituted into the calibration curve to obtain the concentration of the human albumin solution and whether or not the obtained concentration of the human albumin solution corresponded to an actual concentration was confirmed.

Measurement for the amplitude value was performed three times in total. In the three measurements, results for the amplitude value of the direct current component were 123, 124 and 122 and values for the concentration of the human albumin solution, obtained by calculation from the calibration curve were 15.3, 15.5 and 15.2 mg/dl. This indicates that estimated values close to actual concentrations were achieved. Therefore, it has been confirmed that the method using an amplitude value of a direct current component is effective as means for quantifying the concentration of a substance to be measured.

From the above-described results, it has been confirmed that the immunoassay method and immunoassay system of the present invention are effective in detection of the zone phenomenon. Moreover, the present invention allows detection of the zone phenomenon with a single wavelength and a single measurement, so that a plurality of measurements are not necessary and a plurality of reaction vessels do not have to be prepared. It has been also confirmed that multiple wavelength measurement is not required and thus measurement steps and a system configuration become simple. Moreover, it has been confirmed that, according to the present invention, the concentration of a substance to be measured can be measured.

### (Example 3)

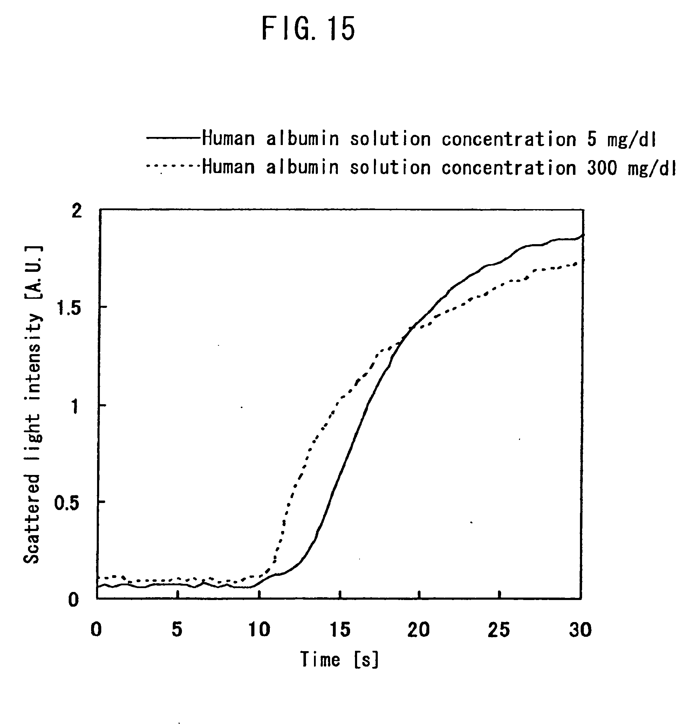

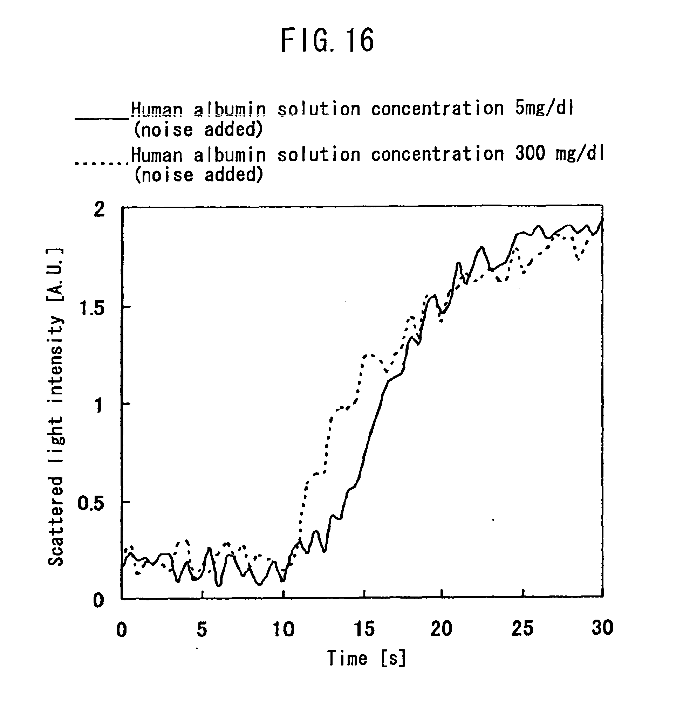

In this example, it will be described that the immunoassay method of this embodiment is hardly influenced by noise. FIG. **15** is a graph showing changes with time in scatted light intensity in human albumin solutions having concentrations of 5 mg/dl and 300 mg/dl obtained in Example 2. In Example 2, discrete Fourier transform was performed to the measurement data in the time interval shown in FIG. **15**, thereby obtaining the amplitude spectrum distribution. On the other hand, in FIG. **16**, random noise of which the fluctuation margin corresponds to 0.2 in terms of scatted light intensity value is added to the measurement data in the time interval shown in FIG. **15**. In this example, using data shown in FIGS. **15** and **16,** the known method using differentiation (difference) and the immunoassay method of the present invention will be compared to each other.

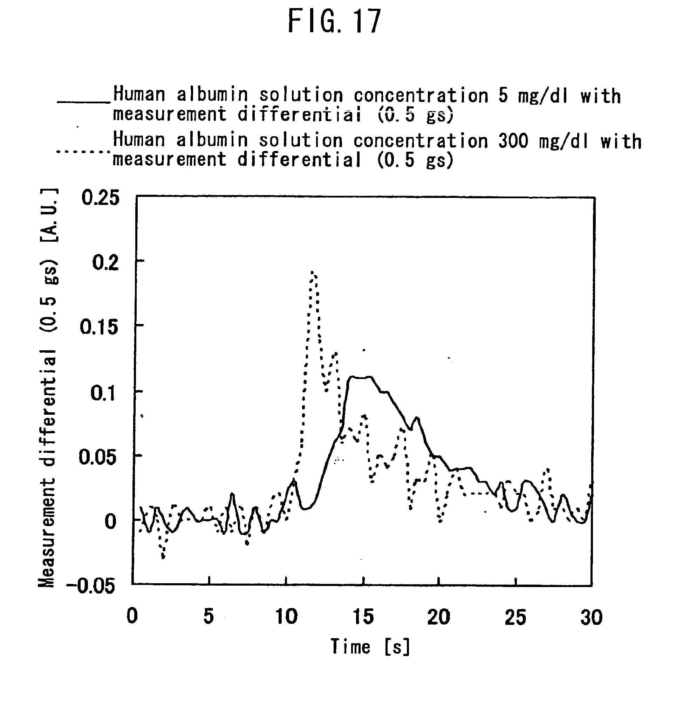

FIG. **17** is a graph showing results obtained from differentiation performed with measurement data in the case where the random noise of FIG. **15** was not added shifted by 0.5 seconds. According to the results, peak positions, i.e., peak positions of reaction rate are different between the human albumin solution having a human albumin concentration of 5 mg/dl in which the zone phenomenon does not occur and the human albumin solution having a human albumin concentration of 300 mg/dl in which the zone phenomenon occurs. Therefore, it can be seen that whether or not the zone phenomenon has occurred can be judged based on FIG. **17**.

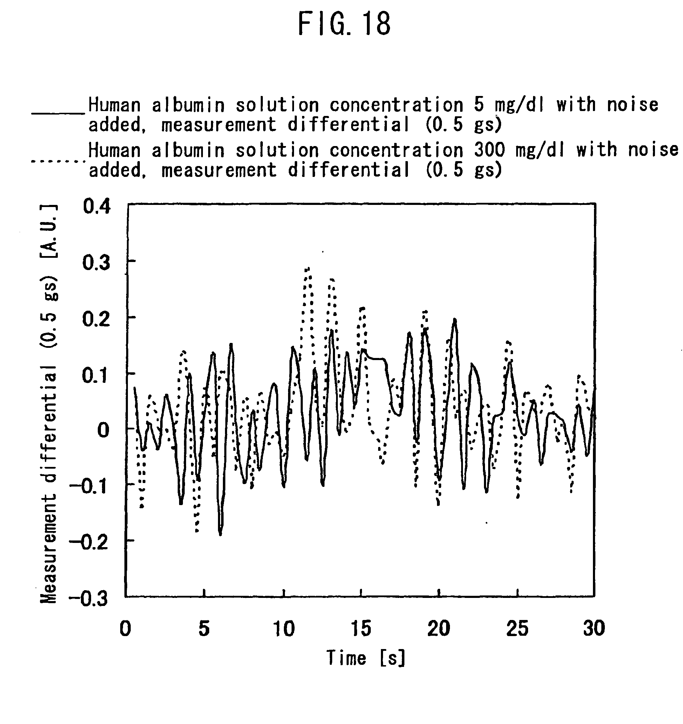

FIG. **18** is a graph showing results obtained from differentiation performed with measurement data in the case where the random noise of FIG. **16** was not added shifted by 0.5 seconds. According to the results, it is not easy to determine a peak position, i.e., a peak position of a reaction rate due to influence of noise. Moreover, differences between the results for the human albumin solution having a human albumin concentration of 5 mg/dl in which the zone phenomenon does not occur and the results for the human albumin solution having a human albumin concentration of 300 mg/dl in which the zone phenomenon occurs are not clear. Therefore, it can be seen that it is not easy to judge based on FIG. **18** whether or not the zone phenomenon has occurred. As has been described, the method based on differentiation (difference) is easily influenced by noise.

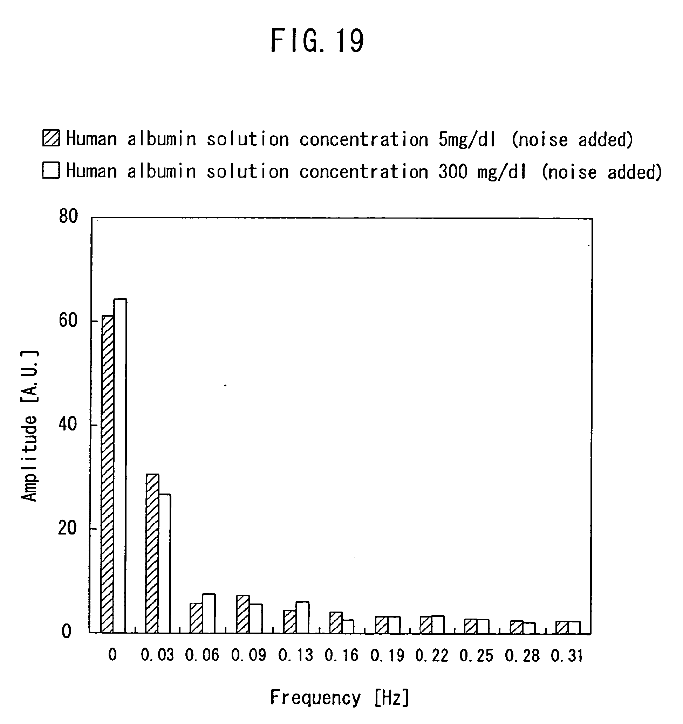

Next, a method for detecting the zone phenomenon according to the present invention will be described. FIG**. 14** is a graph showing results obtained by performing discrete Fourier transform to measurement data to obtain an amplitude distribution when random noise was not added as shown in FIG. **15**, and FIG **19** is a graph showing results obtained by performing discrete Fourier transform to measurement data of FIG. **16** in the case where random noise was added to obtain an amplitude distribution. As clearly shown in each of FIGS. **14** and **19**, the value of {(an amplitude value at 0.0938 Hz) - (an amplitude at 0.0625 Hz)} was not influenced and was positive in the case of the human albumin solution having a human albumin concentration of 5 mg/dl and negative in the case of the human albumin solution having a human albumin concentration of 300 mg/dl. This shows that whether or not the zone phenomenon has occurred can be judged.

From the above-described results, it has been confirmed that according to the immunoassay method of the present invention, results for judgment of whether or not the zone phenomenon has occurred are hardly influenced and can be stably obtained.

### (Example 4)

- Hereinafter, an immunoassay method using the first immunoassay method (in which the time interval **d**_{**1**} + **d**_{**2**} is selected) of the first and second immunoassay methods described in the above-described embodiment will be described.

The configuration of the measurement means **11** is the same as that in Example 1. The measurement data processing means **12** is formed so as to have the following configuration. In this example, a time interval to be selected is set within a range from a time when a substance to be measured and a specific binding substance are mixed and a reaction is started to a time when a certain time period has elapsed after the reaction has been substantially saturated. For the same antigen-antibody reaction described in Example 2, sampling data was obtained from 64 points in a time interval from a start of a reaction to about 32 seconds after the start of the reaction. The obtained data was then assumed to be a periodic signal with a period of 32 seconds and a frequency of 0.03125 Hz and is discrete Fourier transformed on Microsoft Excel (available from Microsoft), thereby obtaining an amplitude spectrum distribution.

For judgment means in the judgment/quantification means **13**, adopted is a method in which at least for an amplitude value at a frequency corresponding to the reciprocal of the time interval **(d**_{**2**} + **d**_{**3**} in FIG **2A**) when a substance to be measured and a specific binding substance are mixed and a reaction is started to a time when a certain time period has elapsed after the reaction has been substantially saturated, the ratio of the amplitude value to the amplitude value of a direct current is obtained and then judgment is performed based on the ratio.

Specifically, the ratio of an amplitude value at a frequency of 0.0625 Hz corresponding to the reciprocal of the time interval **d**_{**2**} + **d**_{**3**}**,** i.e., about 15 seconds to the amplitude value of a direct current component is obtained by calculation. In this manner, different distributions exhibiting by obtained values differ between the case where a substance to be measured has a concentration which does not cause the zone phenomenon and the case where the substance to be measured has a concentration which causes the zone phenomenon, so that differences between the cases are clearly shown. For example, under the conditions for the antigen-antibody reaction described in Example 2, the ratio of the amplitude value at a frequency 0.0625 Hz to the amplitude of the direct current component is 0.135 for the results with respect to the human albumin solution having a human albumin concentration of 5 mg/dl and 0.124 for the results with respect to the human albumin solution having a human albumin concentration of 10 mg/dl, 0.125 for the results with respect to the human albumin solution having a human albumin concentration of 20 mg/dl, 0.123 for the results with respect to the human albumin solution having a human albumin concentration of 30 mg/dl, 0.133 for the results with respect to the human albumin solution having a human albumin concentration of 50 mg/dl, 0.113 for the results with respect to the human albumin solution having a human albumin concentration of 100 mg/dl, 0.110 for the results with respect to the human albumin solution having a human albumin concentration of 200 mg/dl, and 0.095 for the results with respect to the human albumin solution having a human albumin concentration of 300 mg/dl. Thus, the ratio of the amplitude value at a frequency of 0.0625 Hz to the amplitude value of the direct current component exhibits distribution over a value of 0.120 when the concentration of the substance to be measured is a concentration which does not cause the zone phenomenon and it exhibits a distribution below a value of 0.120 when the concentration of the substance to be measured is a concentration which causes the zone phenomenon.

Therefore, under the conditions for the antigen-antibody reaction described in Example 2, with a reference value of 0.120, a difference between the reference value and the ratio of the amplitude value obtained by calculation for a sample solution of which the concentration is not known to the amplitude value of the direct current component, i.e., {(the ratio of the amplitude value at 0.0625 Hz to the amplitude value of the direct current) - (reference value)} is obtained. If the obtained value is positive, it is judged that the known concentration is not the zone region, and if the obtained value is negative, it is judged that the known concentration is the zone region. With this configuration, whether or not the concentration of a substance to be measured is a concentration which does not cause the zone phenomenon can be judged.

From the above-described results, it has been confirmed that the immunoassay method and immunoassay system according to the fourth example are effective in detection of the zone phenomenon. Moreover, the present invention allows detection of the zone phenomenon with a single wavelength and a single measurement, so that a plurality of measurements are not necessary and a plurality of reaction vessels do not have to be prepared. It has been also confirmed that multiple wavelength measurement is not required and thus measurement steps and a system configuration become simple.

## Claims

1. An immunoassay method for measuring a concentration of a substance to be measured and contained in a sample solution, the method comprising the steps of:
a) mixing the sample solution and a specific binding substance capable of specifically binding to the substance to be measured and obtaining, with a predetermined sampling interval, a change with time in an optical change amount in a reaction of the substance to be measured and the specific binding substance as discrete measurement data;
b) selecting a time interval of the measurement data including a time when an increase rate of the optical change amount is maximum;
c) performing, with the time interval assumed to be a period of a periodic function, discrete Fourier transform to the measurement data within the time interval to obtain an amplitude spectrum distribution; and
d) judging, based on a shape of the amplitude spectrum distribution, whether or not a zone phenomenon has occurred in the reaction in the step a),
wherein a combination of the substance to be measured and the specific binding substance is a combination of an antigen and an antibody.

2. The immunoassay method of claim 1, wherein the optical change amount is an amount of change in scattered light intensity or an amount of change in transmitted light.

3. The immunoassay method of claim 1, wherein the time interval has been determined beforehand based on measurement data for the optical change amount measured in a reaction in which no zone phenomenon occurs.

4. The immunoassay method of claim 1, wherein the time interval is from a time when the specific binding substance and the sample solution are mixed to a time when the optical change amount substantially becomes constant after the reaction has been substantially saturated.

5. The immunoassay method of claim 4, wherein in the step d), whether or not the zone phenomenon has occurred in the reaction in the step a) is judged, based on the shape of the amplitude spectrum distribution and a shape of the amplitude spectrum distribution measured in a reaction in which no zone phenomenon occurs.

6. The immunoassay method of claim 4, wherein in the step d), whether or not the zone phenomenon has occurred in the reaction in the step a) is judged, based on a ratio of an amplitude value at a frequency corresponding to a reciprocal of the time interval to a direct current component of the amplitude spectrum distribution.

7. The immunoassay method of claim 6, wherein in the step d), it is judged that no zone phenomenon has occurred in the reaction in the step a) if the ratio is higher than a reference value obtained from respective distributions of a reaction in which no zone phenomenon occurs and a reaction in which the zone phenomenon occurs, and the zone phenomenon has occurred in the reaction in the step a) if the ratio is lower than the reference value.

8. The immunoassay method of claim 1, further comprising, before the step a), the step e) of constructing a reaction system including only one of the sample solution and the specific bonding substance and obtaining, with a predetermined sampling interval, the change with time in the optical change amount in the reaction system as discrete measurement data,
wherein in the step a), the other one of the sample solution and the specific bonding substance which is not contained in the reaction system is added to the reaction system to mix the sample solution and the specific binding substance, and
wherein the time interval is from a time before the specific binding substance and the sample solution are mixed to a time when the optical change amount substantially becomes constant after the reaction has been substantially saturated.

9. The immunoassay method of claim 8, wherein in the step d), it is judged that no zone phenomenon has occurred in the reaction system in the step a) if a difference between an amplitude value at a frequency corresponding to a reciprocal of a time interval from a time when the specific binding substance and the sample solution are mixed to a time when a reaction in the reaction system is substantially saturated and an amplitude value at a frequency corresponding to a reciprocal of a time interval from the time when the specific binding substance and the sample solution are mixed to a time when the optical change amount substantially becomes constant after the reaction in the reaction system has been saturated is a positive number, and the zone phenomenon has occurred in the reaction system in the step a) if the difference is a negative number.

10. The immunoassay method of claim 1, wherein a concentration of the substance to be measured is quantified, based on an amplitude value of a direct current component of the amplitude spectrum distribution in the step c).

## Patentansprüche

1. Ein Immuntest-Verfahren zum Bestimmen einer Konzentration einer zu messenden Substanz, die in einer Probenlösung enthalten ist, wobei das Verfahren die folgenden Schritte umfasst:
a) Mischen der Probenlösung und einer spezifisch bindenden Substanz, die fähig ist, spezifisch an die zu messende Substanz zu binden, und Erhalt, mittels eines vorher bestimmten Probennahme-Intervalls, einer sich mit der Zeit ändernden optischen Größe in einer Reaktion der zu messenden Substanz und der spezifisch bindenden Substanz als diskrete Messwerte;
b) Auswahl eines Zeitintervalls aus den Messwerten, einschließlich einer Zeit, zu der die Steigerungsrate der Änderung der optischen Größe maximal ist;
c) Durchführen einer diskreten Fourier-Transformation der Messwerte mit dem Zeitintervall, von dem angenommen wird, eine Periode einer periodischen Funktion zu sein, um eine Amplitudenspektrum-Verteilung zu erhalten; und
d) Bewerten, anhand der Form der Amplitudenspektrum-Verteilung, ob ein Zonen-Phänomen in der Reaktion in Schritt a) eingetreten ist oder nicht, wobei eine Kombination der zu messenden Substanz und der spezifisch bindenden Substanz eine Kombination eines Antigens und eines Antikörpers ist.

2. Der lmmuntest gemäß Anspruch 1, wobei die Änderung der optischen Größe eine Änderung der Menge gestreuter Lichtintensität oder eine Änderung der Menge an transmittiertem Licht ist.

3. Der Immuntest gemäß Anspruch 1, wobei das Zeitintervall im voraus, aufgrund von Messwerten für die Änderung der optischen Größe in einer Reaktion, in der kein Zonen-Phänomen auftritt, bestimmt wird.

4. Der Immuntest gemäß Anspruch 1, wobei das Zeitintervall von einem Zeitpunkt, an dem die spezifisch bindende Substanz und die Probenlösung gemischt werden, bis zu einem Zeitpunkt reicht, an dem die Veränderung der optischen Größe im wesentlichen konstant wird, nachdem die Reaktion im wesentlichen gesättigt ist.

5. Der Immuntest gemäß Anspruch 4, wobei anhand der Amplitudenspektrum-Verteilung und der Form der Amplitudenspektrum-Verteilung, die einer Reaktion gemessen wird, in der kein Zonen-Phänomen auftritt, im Schritt d) beurteilt wird, ob ein Zonen-Phänomen in der Reaktion in Schritt a) aufgetreten ist oder nicht.

6. Der Immuntest gemäß Anspruch 4, wobei anhand des Verhältnisses zwischen einem Amplitudenwert bei einer Frequenz, die einem reziproken Wert eines Zeitintervalls entspricht, und einer Gleichstromkomponente der Amplitudenspektrum-Verteilung, in Schritt d) beurteilt wird, ob ein Zonen-Phänomen in der Reaktion in Schritt a) aufgetreten ist.

7. Der Immuntest gemäß Anspruch 6, wobei im Schritt d) beurteilt wird, dass kein Zonen-Phänomen in der Reaktion im Schritt a) eingetreten ist, wenn das Verhältnis höher als ein Referenzwert ist, der von den jeweiligen Verteilungen einer Reaktion, in der kein Zonen-Phänomen auftritt, und einer Reaktion, in der das Zonen-Phänomen auftritt, erhalten wird, und beurteilt wird, dass das Zonen-Phänomen in der Reaktion von Schritt a) aufgetreten ist, wenn das Verhältnis niedriger als der Referenzwert ist.

8. Der Immuntest gemäß Anspruch 1, weiter umfassend, vor Schritt a), den Schritt e) des Aufbaus eines Reaktionssystems, das nur einen Vertreter aus der Gruppe der Probenlösung und der spezifisch bindenden Substanz einschließt, und des Erhalts, mittels eines vorher bestimmten Probennahme-Intervalls, einer sich mit der Zeit ändernden optischen Größe in dem Reaktionssystem als diskrete Messwerte, wobei in Schritt a) der andere Vertreter aus der Gruppe der Probenlösung und der spezifisch bindenden Substanz, der nicht im Reaktionssystem enthalten ist, dem Reaktionssystem zugefügt wird, um die Probenlösung und die spezifisch bindende Substanz zu mischen, und wobei das Zeitintervall von einem Zeitpunkt, der vor dem Mischen der spezifisch bindenden Substanz und der Probenlösung liegt, bis zu einem Zeitpunkt reicht, an dem die Änderung der optischen Größe im wesentlichen konstant wird, nachdem die Reaktion im wesentlichen gesättigt ist.

9. Der Immuntest gemäß Anspruch 8, wobei im Schritt d) beurteilt wird, dass kein Zonen-Phänomen in dem Reaktionssystem in Schritt a) eingetreten ist, wenn eine Differenz zwischen einem Amplitudenwert bei einer Frequenz, die dem reziproken Wert eines Zeitintervalls entspricht, das von einer Zeit, zu der die spezifisch bindende Substanz und die Testlösung gemischt werden, bis zu einer Zeit reicht, zu der eine Reaktion in dem Reaktionssystem im wesentlichen gesättigt ist, und einem Amplitudenwert bei einer Frequenz, die dem reziproken Wert eines Zeitintervalls entspricht, das von einer Zeit, zu der die spezifisch bindende Substanz und die Probenlösung gemischt werden, bis zu einer Zeit reicht, zu der die Änderung der optischen Größe im wesentlichen konstant wird, nachdem die Reaktion im Reaktionssystem gesättigt ist, eine positive Zahl ist und beurteilt wird, dass das Zonen-Phänomen in dem Reaktionssystem in Schritt a) eingetreten ist, wenn die Differenz eine negative Zahl ist.

10. Der lmmuntest gemäß Anspruch 1, wobei anhand eines Amplitudenwertes einer Gleichstromkomponente der Amplitudenspektrum-Verteilung in Schritt c) eine Konzentration der zu messenden Substanz quantifiziert wird.

## Revendications

1. Procédé d'immunoessai destiné à mesurer une concentration d'une substance à mesurer et contenue dans une solution d'échantillon, le procédé comprenant les étapes consistant à :
a) mélanger la solution d'échantillon et une substance de liaison spécifique capable de se lier spécifiquement à une substance pour être mesurée et obtenir, avec un intervalle d'échantillonnage prédéterminé, un changement avec le temps dans une quantité de changement optique dans une réaction de la substance à mesurer et de la substance de liaison spécifique en tant que données de mesurage discrètes ;
b) choisir un intervalle de temps des données de mesurage incluant un moment où un taux d'accroissement de la quantité de changement optique est maximum ;
c) réaliser, avec l'intervalle de temps supposé être une période d'une fonction périodique, une transformation de Fourier discrète pour les données de mesurage à l'intérieur de l'intervalle de temps pour obtenir une distribution de spectre d'amplitude ; et
d) juger, sur la base d'une forme de la distribution du spectre d'amplitude, si oui ou non un phénomène de zone s'est produit dans la réaction de l'étape a),
dans lequel une combinaison de la substance à mesurer et de la substance de liaison spécifique est une combinaison d'un antigène et d'un anticorps.

2. Procédé d'immunoessai selon la revendication 1, dans lequel la quantité de changement optique est une quantité de changement d'intensité de lumière diffusée ou une quantité de changement de lumière transmise.

3. Procédé d'immunoessai selon la revendication 1, dans lequel l'intervalle de temps a été déterminé préalablement sur la base de données de mesurage pour la quantité de changement optique mesurée dans une réaction dans laquelle aucun phénomène de zone ne se produit.

4. Procédé d'immunoessai selon la revendication 1, dans lequel l'intervalle de temps va d'un moment où la substance de liaison spécifique et la solution d'échantillon sont mélangées jusqu'à un moment où la quantité de changement optique devient constante après que la réaction ait été substantiellement saturée.

5. Procédé d'immunoessai selon la revendication 4, dans lequel au cours de l'étape d), si oui ou non le phénomène de zone s'est produit lors de la réaction de l'étape a) est jugé, sur la base de la forme de la distribution du spectre d'amplitude et d'une forme de la distribution du spectre d'amplitude mesurée dans une réaction dans laquelle aucun phénomène de zone ne s'est produit.

6. Procédé d'immunoessai selon la revendication 4, dans lequel au cours de l'étape d), si oui ou non le phénomène de zone s'est produit lors de la réaction de l'étape a) est jugé, sur la base d'un rapport d'une valeur d'amplitude à une fréquence correspondant à une réciproque de l'intervalle de temps pour une composante de courant continu de la distribution de spectre d'amplitude.

7. Procédé d'immunoessai selon la revendication 6, dans lequel au cours de l'étape d), il est jugé qu'aucun phénomène de zone ne s'est produit lors de la réaction de l'étape a) si le rapport est supérieur à une valeur de référence obtenue à partir de distributions respectives d'une réaction dans laquelle aucun phénomène de zone ne se produit et d'une réaction dans laquelle le phénomène de zone se produit, et que le phénomène de zone s'est produit dans la réaction de l'étape a) si le rapport est inférieur à la valeur de référence.

8. Procédé d'immunoessai selon la revendication 1, comprenant en outre, avant l'étape a), l'étape e) consistant à construire un système de réaction n'incluant que l'une parmi la solution d'échantillon et la substance de liaison spécifique et à obtenir, avec un intervalle d'échantillonage prédéterminé, le changement dans le temps concernant une quantité de changement optique dans le système de réaction en tant que données de mesurage discrètes,
dans lequel lors de l'étape a), l'autre parmi la solution d'échantillon et la substance de liaison spécifique qui n'est pas contenue dans le système de réaction est ajoutée au système de réaction pour mélanger la solution d'échantillon et la substance de liaison spécifique, et
dans lequel l'intervalle de temps va d'un moment situé avant que la substance de liaison spécifique et la solution d'échantillon ne soient mélangées jusqu'à un moment où la quantité de changement optique devient constante après que la réaction ait été sensiblement saturée.

9. Procédé d'immunoessai selon la revendication 8,
dans lequel au cours de l'étape d), il est jugé qu'aucun phénomène de zone ne s'est produit dans le système de réaction de l'étape a) si une différence entre une valeur d'amplitude à une fréquence correspondant à une réciproque d'un intervalle de temps allant d'un moment où la substance de liaison spécifique et la solution d'échantillon sont mélangées jusqu'à un moment où une réaction du système de réaction est sensiblement saturée et une valeur d'amplitude à une fréquence correspondant à une réciproque d'un intervalle de temps allant d'un moment où la substance de liaison spécifique et la solution d'échantillon sont mélangées jusqu'à un moment où la quantité de changement optique devient constante après que la réaction du système de réaction ait été saturée est un nombre positif, et que le phénomène de zone s'est produit dans le système de réaction au cours de l'étape a) si la différence est un nombre négatif.

10. Procédé d'immunoessai selon la revendication 1, dans lequel une concentration de la substance à mesurer est quantifiée, sur la base d'une valeur d'amplitude d'une composante de courant continu de la distribution de spectre d'amplitude de l'étape c).
